# EUROPÄISCHE PATENTSCHRIFT

(11) **EP 1 523 475 B1**
(45) Veröffentlichungstag und Bekanntmachung des Hinweises auf die Patenterteilung: **23.12.2009**
(21) Anmeldenummer: 03763692.5
(22) Anmeldetag: 03.07.2003
(51) Int. Cl.: C07D 211/62, C07D 249/08, C07D 257/04, C07D 271/10, C07D 253/06, C07D 231/26, C07D 307/68, C07D 235/18, A61K 31/17, A61P 3/10

(54) **HETEROZYKLISCH SUBSTITUIERTE BENZOYLHARNSTOFFE, VERFAHREN ZU IHRER HERSTELLUNG UND IHRE VERWENDUNG ALS ARZNEIMITTEL**
HETEROCYCLICALLY SUBSTITUTED BENZOYLUREAS, METHOD FOR THEIR PRODUCTION AND THEIR USE AS MEDICAMENTS
BENZOYL UREES A SUBSTITUTION HETEROCYCLIQUE, PROCEDES POUR LEUR PRODUCTION ET LEUR UTILISATION COMME MEDICAMENTS

(30) Priorität: 12.07.2002 DE 10231627; 17.02.2003 DE 10306503; 06.05.2003 DE 10320326
(43) Veröffentlichungstag der Anmeldung: 20.04.2005
(73) Patentinhaber: Sanofi-Aventis Deutschland GmbH, 65929 Frankfurt am Main (DE)
(72) Erfinder: SCHOENAFINGER, Karl, 63755 Alzenau (DE); DEFOSSA, Elisabeth, 65510 Idstein (DE); KADEREIT, Dieter, 63071 Offenbach (DE); VON ROEDERN, Erich, 65795 Hattersheim (DE); KLABUNDE, Thomas, 65929 Frankfurt (DE); BURGER, Hans-Joerg, Morristown, NJ 07960 (US); HERLING, Andreas, 65520 Bad Camberg (DE); WENDT, Karl-Ulrich, 65926 Frankfurt am Main (DE)
(86) Internationale Anmeldenummer: PCT/EP2003/007078
(87) Internationale Veröffentlichungsnummer: WO 2004/007455

(56) Entgegenhaltungen:
- EP-A- 0 193 249
- EP-A- 0 242 322
- WO-A-01/94300
- WO-A-02/096864
- DE-A- 10 116 768

## Beschreibung

Die Erfindung betrifft heterozyklisch substituierte Benzoylharnstoffe sowie deren physiologisch verträgliche Salze und physiologisch funktionelle Derivate.

Es sind bereits heterozyklisch substituierte Benzoylharnstoffe mit pestizider Wirkung im Stand der Technik beschrieben (EP 0 242 322).

Der Erfindung lag die Aufgabe zugrunde, Verbindungen zur Verfügung zu stellen, die eine therapeutisch verwertbare Blutzucker senkende Wirkung entfalten.

Die Erfindung betrifft daher Verbindungen der Formel I, 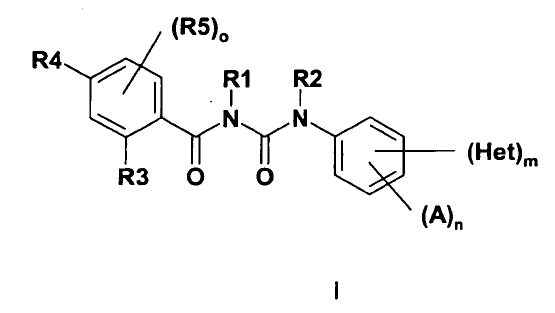 worin bedeuten
- R1, R2: unabhängig voneinander H, (C₁-C₆)-Alkyl, wobei Alkyl mit OH, O-(C₁-C₄)-Alkyl, NH₂, NH-(C₁-C₄)-Alkyl oder N-[(C₁-C₆)-Alkyl]₂, substituiert sein kann, O-(C₁-C₆)-Alkyl, CO-(C₁-C₆)-Alkyl, COO-(C₁-C₆)-Alkyl, (C₁-C₆)-Alkylen-COOH oder (C₁-C₆)-Alkylen-COO-(C₁-C₆)-Alkyl;
- R3, R4: unabhängig voneinander F, Cl, Br, OH, NO₂, CN, (C₁-C₆)-Alkyl, O-(C₁-C₄)-Alkyl, O-(C₂-C₆)-Alkenyl oder (C₂-C₆)-Alkinyl, wobei Alkyl, Alkenyl und Alkinyl mehrfach durch F, Cl oder Br substituiert sein können;
- R5: H, F, Cl, Br, OH, NO₂, CN, (C₁-C₆)-Alkyl, O-(C₁-C₄)-Alkyl, CO-(C₁-C₆)-Alkyl, (C₀-C₆)-Alkylen-COOH, (C₀-C₆)-Alkylen-COO-(C₁-C₆)-Alkyl, SO₂-(C₁-C₆)-Alkyl, O-(C₂-C₆)-Alkenyl oder (C₂-C₆)-Alkinyl, wobei Alkyl, Alkenyl und Alkinyl mehrfach durch F, Cl oder Br substituiert sein können;
- A: H, F, Cl, Br, OH, NO₂, CN, (C₁-C₆)-Alkyl, CO-(C₁-C₆)-Alkyl, (C₀-C₆)-Alkylen-COOH, (C₀-C₆)-Alkylen-COO(C₁-C₆)-Alkyl, SO₂-(C₁-C₆)-Alkyl, (C₂-C₆)-Alkenyl, (C₂-C₆)-Alkinyl, O-(C₁-C₆)-Alkyl, S(O)₁₋₂-(C₁-C₆)-Alkyl-, NH-(C₁-C₆)-Alkyl, N-((C₁-C₆)-Alkyl)₂, COOH, COO-(C₁-C₆)-Alkyl, CONH₂, CONH-(C₁-C₆)-Alkyl, CON-((C₁-C₆)-Alkyl)₂, SO₂NH₂, SO₂NH-(C₁-C₆)-Alkyl, SO₂N-((C₁-C₆)-Alkyl)₂ oder NHCOR6, wobei Alkyl, Alkenyl und Alkinyl mehrfach durch F, Cl, Br, COOH, COO-(C₁-C₆)-Alkyl, CONH₂, CONH-(C₁-C₆)-Alkyl, CON-((C₁-C₆)-Alkyl)₂ oder OCO-(C₁-C₆)-Alkyl substituiert sein können;
- R6: H, (C₁-C₆)-Alkyl, (C₃-C₇)-Cycloalkyl, (C₃-C₇)-Cycloalkyl-(C₁-C₄)-alkylen, (C₂-C₆)-Alkenyl, (C₂-C₆)-Alkinyl, (C₁-C₆)-Alkylen-COO-(C₁-C₆)-alkyl, (C₁-C₆)-Alkylen-CO-(C₁-C₆)-alkyl, (C₀-C₆)-Alkylen-COOH, , (C₁-C₆)-Alkylen-CONH₂, (C₆-C₁₀)-Aryl, (C₁-C₄)-Alkylen-(C₆-C₁₀)-aryl, Heteroaryl, (C₁-C₄)-Alkylen-heteroaryl oder CO-Heteroaryl, wobei Alkyl, Cycloalkyl, Alkylen, Alkenyl und Alkinyl mehrfach mit F, Cl, Br, O-(C₁-C₄)-Alkyl), COO-(C₁-C₄-Alkyl) oder N-((C₁-C₄)-Alkyl)₂ und wobei Aryl und Heteroaryl mehrfach mit F, Cl, Br, NO₂, CN, O-(C₁-C₄)-Alkyl), 5-COO-(C₁-C₄-Alkyl), N-((C₁-C₄)-Alkyl)₂ oder (C₁-C₆)-Alkyl substituiert sein können;
- n: 0, 1, 2 oder 3;
- m: 1, 2, 3, 4 oder 5;
- o: 0, 1, 2 oder 3;
- Het: heterozyklischer 4- bis 7-gliedrigen Ring, der bis zu 4 Heteroatome N, O oder S als Ringglieder enthalten kann, wobei Pyrrol ausgenommen ist und wobei der heterozyklische Ring substituiert sein kann mit R7, R8 und R9;
- R7, R8, R9: unabhängig voneinander H, F, Cl, Br, (C₁-C₆)-Alkyl, O-(C₁-C₆)-Alkyl, O-(C₂-C₆)-Alkenyl, O-(C₂-C₆)-Alkinyl, OH, Oxo, O-(C₁-C₆)-Alkyl, NH₂, NH-(C₁-C₆)-Alkyl, N-((C₁-C₆)-Alkyl)₂, COOH, CO-(C₁-C₆)-Alkyl, COO-(C₁-C₆)-Alkyl, CONH₂, CONH-(C₁-C₆)-Alkyl, CON-((C₁-C₆)-Alkyl)₂, (C₀-C₆)-Alkylen-Aryl oder (C₁-C₆)-Alkylen-COO-(C₁-C₆)-Alkyl; worin Alkyl, Alkenyl, Alkinyl, Alkylen und Aryl durch COOH, CONH₂, CONH-(C₁-C₆)-Alkyl; CON-((C₁-C₆)-Alkyl)₂, OCO-(C₁-C₆)-Alkyl, F, Cl, (C₁-C₆)-Alkyl oder O-(C₁-C₆)-Alkyl substituiert sein können;
- und worin 2: der Reste R7, R8 und R9 gemeinsam einen an Het ankondensierten Ring bilden können;
sowie deren physiologisch verträgliche Salze.

Weiterhin ganz bevorzugt sind die Verbindungen der Formel Iaa, 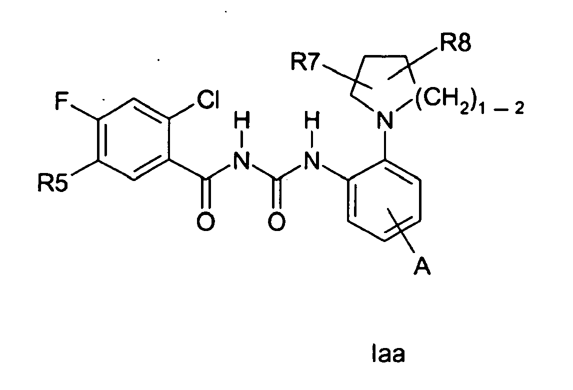 worin bedeuten
- R5: H, F;
- A: H, F, Cl, (C₁-C₆)-Alkyl, CF₃, COO-(C₁-C₆)-Alkyl, COOH, SO₂-(C₁-C₆)-Alkyl;
- R7: H, Phenyl;
- R8: -(C=O)-X
- X: OH, O-(C₁-C₆)-Alkyl, NH₂, NH-(C₁-C₆)-Alkyl, N-((C₁-C₆)-Alkyl)₂;
sowie deren physiologisch verträgliche Salze.

Die Erfindung bezieht sich auf Verbindungen der Formel I, in Form ihrer Racemate, racemischen Mischungen und reinen Enantiomere sowie auf ihre Diastereomere und Mischungen davon.

Die Alkylreste in den Substituenten A, R1, R2, R3, R4, R5, R6, R7, R8 und R9 können sowohl geradkettig wie verzweigt sein. Können Reste oder Substituenten mehrfach in den Verbindungen der Formel I auftreten, so können sie alle unabhängig voneinander die angegebenen Bedeutungen haben und gleich oder verschieden sein.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze), Erdalkalisalze (wie Magnesium- und Calciumsalze), Trometamol (2-Amino-2-hydroxymethyl-1,3-propandiol), Diethanolamin, Lysin oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen, wie zum Beispiel in H. Okada et al., Chem. Pharm. Bull. 1994, 42, 57-61 beschrieben. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I wie vorstehend beschrieben, sowie ihre Salze, Solvate und physiologisch funktionellen Derivate wie hierin beschrieben.

Unter einem Arylrest wird ein Phenyl, Naphthyl-, Biphenyl-, Tetrahydronaphthyl-, alpha- oder beta-Tetralon-, Indanyl- oder Indan-1-on-ylrest verstanden.

Die Verbindung(en) der Formel (I) können auch in Kombination mit weiteren Wirkstoffen verabreicht werden.

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg und 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Poylvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägem, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglykole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Als weitere Wirkstoffe für die Kombinationspräparate sind geeignet:

Alle Antidiabetika, die in der Roten Liste 2001, Kapitel 12 genannt sind. Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.
Antidiabetika umfassen Insulin und Insulinderivate, wie z.B. Lantus^{®} (siehe www.lantus.com) oder HMR 1964, schnell wirkende Insuline (siehe US 6,221,633), GLP-1-Derivate wie z.B. diejenigen die in WO 98/08871 von Novo Nordisk A/S offenbart wurden, sowie oral wirksame hypoglykämische Wirkstoffe. Die oral wirksamen hypoglykämischen Wirkstoffe umfassen vorzugsweise Sulphonylfharnstoffe, Biguanidine, Meglitinide, Oxadiazolidindione, Thiazolidindione, Glukosidase-Inhibitoren, Glukagon-Antagonisten, GLP-1-Agonisten, Kaliumkanalöffner, wie z.B. diejenigen, die in WO 97/26265 und WO 99/03861 von Novo Nordisk A/S offenbart wurden, Insulin-Sensitizer, Inhibitoren von Leberenzymen, die an der Stimulation der Glukoneogenese und/oder Glykogenolyse beteiligt sind, Modulatoren der Glukoseaufnahme, den Fettstoffwechsel verändernde Verbindungen wie antihyperlipidämische Wirkstoffe und antilipidämische Wirkstoffe, Verbindungen, die die Nahrungsmitteleinnahme verringern, PPAR- und PXR-Agonisten und Wirkstoffe, die auf den ATP-abhängigen Kaliumkanal der Betazellen wirken.

Bei einer Ausführungsform der Erfindung werden die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin verabreicht.

Bei einer Ausführungsform der Erfindung werden die Verbindungen der Formel I in Kombination mit einem Cholesterinresorptionsinhibitor, wie z.B. Ezetimibe, Tiqueside, Pamaqueside, verabreicht.

Bei einer Ausführungsform der Erfindung werden die Verbindungen der Formel I in Kombination mit einem PPAR gamma Agonist, wie z.B. Rosiglitazon, Pioglitazon, JTT-501, GI 262570, verabreicht.

Bei einer Ausführungsform der Erfindung werden die Verbindungen der Formel I in Kombination mit PPAR alpha Agonist, wie z.B. GW 9578, GW 7647, verabreicht.

Bei einer Ausführungsform der Erfindung werden die Verbindungen der Formel I in Kombination mit einem gemischten PPAR alpha/gamma Agonisten, wie z.B. GW 1536, AVE 8042, AVE 8134, AVE 0847, oder wie in PCT/US 11833, PCT/US 11490, DE10142734.4 beschrieben verabreicht.

Bei einer Ausführungsform der Erfindung werden die Verbindungen der Formel I in Kombination mit einem Fibrat, wie z.B. Fenofibrat, Clofibrat, Bezafibrat, verabreicht.

Bei einer Ausführungsform der Erfindung werden die Verbindungen der Formel I in Kombination mit einem MTP-Inhibitor, wie z.B. Implitapide , BMS-201038, R-103757, verabreicht.

Bei einer Ausführungsform der Erfindung werden die Verbindungen der Formel I in Kombination mit Gallensäureresorptionsinhibitor (siehe z.B. US 6,245,744 oder US 6,221,897), wie z.B. HMR 1741, verabreicht.

Bei einer Ausführungsform der Erfindung werden die Verbindungen der Formel I in Kombination mit einem CETP-Inhibitor, wie z.B. JTT-705 , verabreicht.

Bei einer Ausführungsform der Erfindung werden die Verbindungen der Formel I in Kombination mit einem polymeren Gallensäureadsorber, wie z.B. Cholestyramin, Colesevelam, verabreicht.

Bei einer Ausführungsform der Erfindung werden die Verbindungen der Formel I in Kombination mit einem LDL-Rezeptorinducer (siehe US 6,342,512), wie z.B. HMR1171, HMR1586, verabreicht.

Bei einer Ausführungsform der Erfindung werden die Verbindungen der Formel I in Kombination mit einem ACAT-Inhibitor, wie z.B. Avasimibe, verabreicht.

Bei einer Ausführungsform der Erfindung werden die Verbindungen der Formel I in Kombination mit einem Antioxidans, wie z.B. OPC-14117, verabreicht.

Bei einer Ausführungsform der Erfindung werden die Verbindungen der Formel I in Kombination mit einem Lipoprotein-Lipase Inhibitor, wie z.B. NO-1886, verabreicht.

Bei einer Ausführungsform der Erfindung werden die Verbindungen der Formel I in Kombination mit einem ATP-Citrat-Lyase Inhibitor, wie z.B. SB-204990, verabreicht.

Bei einer Ausführungsform der Erfindung werden die Verbindungen der Formel I in Kombination mit einem Squalen Synthetase Inhibitor, wie z.B. BMS-188494, verabreicht.

Bei einer Ausführungsform der Erfindung werden die Verbindungen der Formel I in Kombination mit einem Lipoprotein(a) antagonist, wie z.B. CI-1027 oder Nicotinsäure, verabreicht.

Bei einer Ausführungsform der Erfindung werden die Verbindungen der Formel I in Kombination mit einem Lipase Inhibitor, wie z.B. Orlistat, verabreicht.

Bei einer Ausführungsform der Erfindung werden die Verbindungen der Formel I in Kombination mit Insulin verabreicht.
Bei einer Ausführungsform werden die Verbindungen der Formel I in Kombination mit einem Sulphonylharnstoff, wie z.B. Tolbutamid, Glibenclamid, Glipizid oder Glimepirid verabreicht.
Bei einer Ausführungsform werden die Verbindungen der Formel I in Kombination mit einem Biguanid, wie z.B. Metformin, verabreicht.

Bei wieder einer Ausführungsform werden die Verbindungen der Formel I in Kombination mit einem Meglitinid, wie z.B. Repaglinid, verabreicht.
Bei einer Ausführungsform werden die Verbindungen der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]-phenyl]methyl]-2,4-thiazolidindion, verabreicht.
Bei einer Ausführungsform werden die Verbindungen der Formel I in Kombination mit einem α-Glukosidase-Inhibitor, wie z.B. Miglitol oder Acarbose, verabreicht.
Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid. Bei einer Ausführungsform werden die Verbindungen der Formel I in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht.

Bei einer weiteren Ausführungsform werden die Verbindungen der Formel I in Kombination mit CART-Modulatoren (siehe "Cocaine-amphetamine-regulated transcript influences energy metabolism, anxiety and gastric emptying in mice" Asakawa, A, et al., M.:Hormone and Metabolic Research (2001), 33(9), 554-558), NPY-Antagonisten z.B. Naphthalin-1-sulfonsäure {4-[(4-amino-quinazolin-2-ylamino)-methyl]-cyclohexylmethyl}- amid; hydrochlorid (CGP 71683A)), MC4-Agonisten (z.B. 1-Aminö-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid; (WO 01/91752)), Orexin-Antagonisten (z.B. 1-(2-Methyl-benzoxazol-6-yl)-3-[1,5]naphthyridin-4-yl-harnstoff; hydrochloride (SB-334867-A)), H3-Agonisten (3-Cyclohexyl-1-(4,4-dimethyl-1,4,6,7-tetrahydro-imidazo[4,5-c]pyridin-5-yl)-propan-1- on Oxalsäuresalz (WO 00 / 63208)); TNF-Agonisten, CRF-Antagonisten (z.B. [2-Methyl-9-(2,4,6-trimethyl-phenyl)-9H-1,3,9-triaza-fluoren-4-yl]-dipropyl-amin (WO 00/66585)), CRF BP-Antagonisten (z.B. Urocortin), Urocortin-Agonisten, β3-Agonisten (z.B. 1-(4-Chloro-3-methanesulfonylmethyl-phenyl)-2-[2-(2,3-dimethyl-1H-indol-6-yloxy)-ethylamino]-ethanol; hydrochloride (WO 01/83451)), MSH (Melanocyt-stimulierendes Hormon)-Agonisten, CCK-A Agonisten (z.B. {2-[4-(4-Chloro-2,5-dimethoxy-phenyl)-5-(2-cyclohexyl-ethyl)-thiazol-2-ylcarbamoyl]-5,7- dimethyl-indol-1-yl}-acetic acid Trifluoressigsäuresalz (WO 99/15525)); Serotonin-Wiederaufnahme-Inhibitoren (z.B. Dexfenfluramine), gemischte Sertonin- und noradrenerge Verbindungen (z.B. WO 00/71549), 5HT-Agonisten z.B. 1-(3-Ethyl-benzofuran-7-yl)-piperazin Oxalsäuresalz (WO 01/09111), Bombesin-Agonisten, Galanin-Antagonisten, Wachstumshormon (z.B. humanes Wachstumshormon), Wachstumshormon freisetzende Verbindungen (6-Benzyloxy-1-(2-diisopropylamino-ethylcarbamoyl)-3,4-dihydro-1H-isoquinoline-2-carboxylic acid tert-butyl ester (WO 01/85695)), TRH-Agonisten (siehe z.B. EP 0 462 884) entkoppelnde Protein 2- oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881),
DA-Agonisten (Bromocriptin, Doprexin), Lipase/Amylase-Inhibitoren (z.B. WO 00/40569), PPAR-Modulatoren (z.B. WO 00/78312), RXR-Modulatoren oder TR-β-Agonisten verabreicht.

Bei einer Ausführungsform der Erfindung ist der weitere Wirkstoff Leptin; siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform ist der weitere Wirkstoff Dexamphatamin oder Amphetamin. Bei einer Ausführungsform ist der weitere Wirkstoff Fenfluramin oder Dexfenfluramin. Bei noch einer Ausführungsform ist der weitere Wirkstoff Sibutramin.
Bei einer Ausführungsform ist der weitere Wirkstoff Orlistat.
Bei einer Ausführungsform ist der weitere Wirkstoff Mazindol oder Phentermin.

Bei einer Ausführungsform werden die Verbindungen der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties &Food Ingredients GmbH, Industriepark Höchst, 65926 Frankfurt / Main)) verabreicht. Die Kombination mit Caromax^{®} kann in einer Zubereitung erfolgten, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

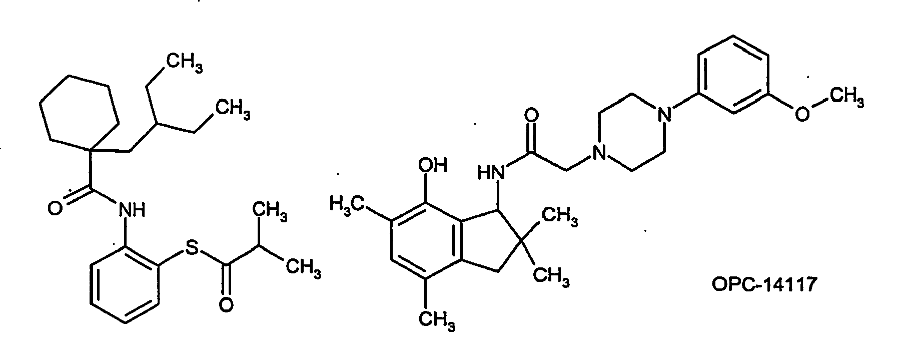

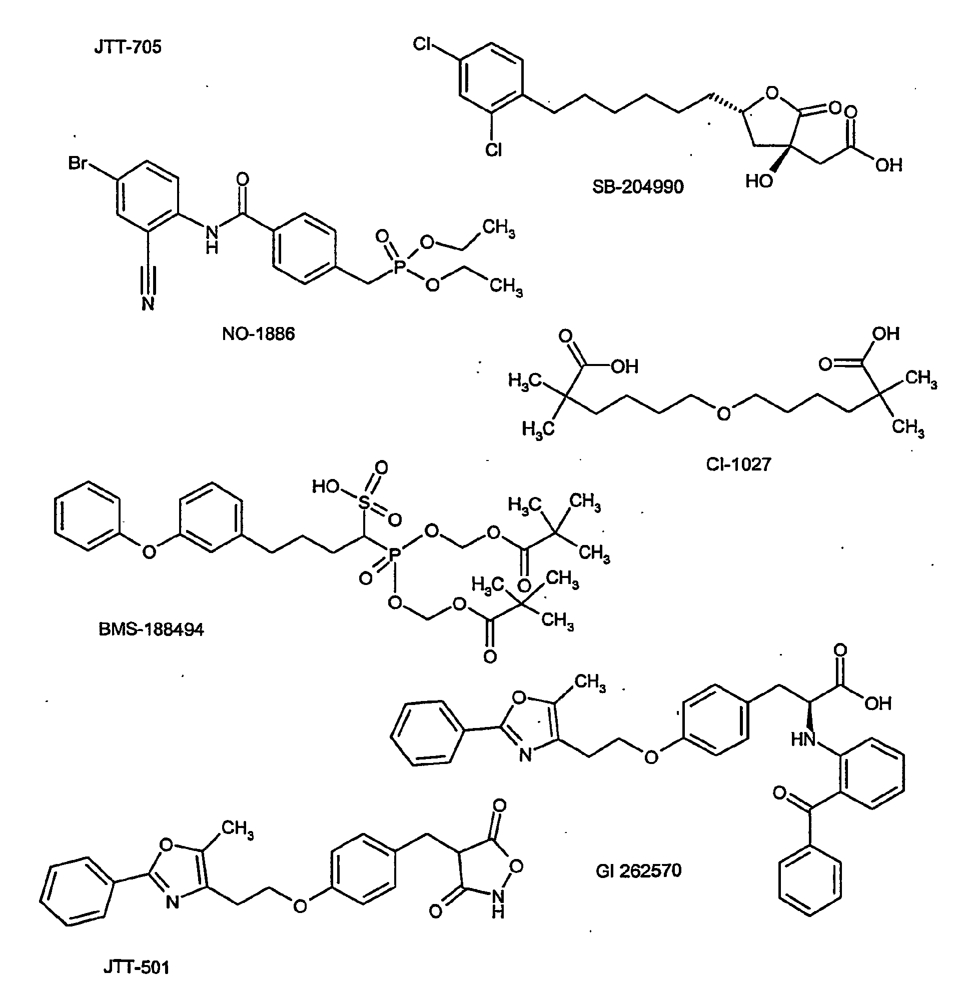

Die nachfolgend aufgeführten Beispiele dienen zur Erläuterung der Erfindung, ohne diese jedoch einzuschränken. Die gemessenen Fest-, bzw. Zersetzungspunkte (Fp.) wurden nicht korrigiert und sind generell von der Aufheizgeschwindigkeit abhängig.

### Beispiel 1:

### a) 1-(3-Fluor-4-nitro-phenyl)-1H-[1,2,4]triazol

Die Mischung bestehend aus 2,5 g 3-Fluor-4-nitro-phenylhydrazin, 1,2 g [1,2,3]Triazin und 50 ml Ethanol wurde 6 Stunden unter Umrühren zum Rückfluss erhitzt. Nach dem Einengen des Reaktionsgemisches im Vakuum wurde der Rückstand säulenchromatografisch aufgearbeitet (LM: Dichlormethan:Methanol = 99:1; Kieselgel).
Ausbeute: 0,8 g Fp.: 99,9 °C

### b) 1-(4-Amino-3-fluor-phenyl)-1H-[1,2,4]triazol

In die Mischung bestehend aus 260 mg 1-(3-Fluor-4-nitrophenyl)-1H-[1,2,4]triazol, 30 mg Pd/C und 30 ml Tetrahydrofuran wurde unter Normaldruck Wasserstoff eingeleitet, bis die theoretische Menge aufgenommen war. Nach dem Absaugen des Katalysators und Einengen des Gemisches am Vakuum wurde der verbleibende ölige Rückstand durch Säulenchromatografie gereinigt (LM: Dichlormethan:Methanol = 98:2; Kieselgel).
Ausbeute: 100 mg Fp.: 93,6 °C

### c) 1-(2-Chlor-4,5-difluor-benzoyl)-3-(2-fluor-4-[1,2,4]triazol-1-yl-phenyl)-harnstoff

Zur Lösung von 75 mg 1-(4-Amino-3-fluor-phenyl)-1H-[1,2,4]triazol in 4 ml Acetonitril wurde die Lösung äquivalenter Mengen von 2-Chlor-4,5-difluor-benzoyl-isocyanat getropft und die Mischung 30 Minuten bei Raumtemperatur gerührt. Der Feststoff wurde abgesaugt und im Vakuum getrocknet.
Ausbeute: 84 mg Fp.: 195,0 °C

### Beispiel 2:

### a) 3-(3-Methoxy-4-nitro-phenyl)-5-methyl-4H-[1,2,4]triazol

Die Mischung bestehend aus 590 mg 3-Methoxy-4-nitro-benzoesäurehydrazid, 6 ml Pyridin und 210 mg Thioacetamid wurde 2 Stunden auf 95 °C erhitzt. Nach dem Erkalten wurden die flüchtigen Anteile bei 40 °C im Vakuum entfernt und der Rückstand einer säulenchromatografischen Reinigung unterworfen (Kieselgel, LM: Dichlormethan:Methanol = 95:5).
Ausbeute: 100 mg Fp.: 176,0 °C

### b) 2-Methoxy-4-(5-methyl-4H-[1,2,4]triazol-3-yl)-phenylamin

wurde durch Hydrierung von 100 mg 3-(3-Methoxy-4-nitro-phenyl)-5-methyl-4H-[1,2,4]triazol in Gegenwart von Pd/C in THF hergestellt und ohne weitere Reinigung weiter verwendet.
Ausbeute: 110 mg (roh) Fp.: 76,9 °C

### c) 1-(2-Chlor-4-fluor-benzoyl)-3-[2-methoxy-4-(5-methyl-4H-[1,2,4]triazol-3-yl)-phenyl]-harnstoff

Zur Lösung von 35 mg 2-Methoxy-4-(5-methyl-4H-[1,2,4]triazol-3-yl)-phenylamin in 3 ml Acetonitril wurde die Lösung äquivalenter Mengen von 2-Chlor-4-fluor-benzoyl-isocyanat getropft und die Mischung 30 Minuten bei Raumtemperatur gerührt. Der Feststoff wurde dann abgesaugt, mit t-Butyl-methylether verrührt, abgesaugt und im Vakuum getrocknet.
Ausbeute: 33 mg Fp.: 269,1 °C

### Beispiel 3:

### 1-(2-Chlor-4,5-difluor-benzoyl)-3-[2-methoxy-4-(5-methyl-4H-[1,2,4]triazol-3-yl)-phenyl]-harnstoff

Zur Lösung von 30 mg 2-Methoxy-4-(5-methyl-4H-[1,2,4]triazol-3-yl)-phenylamin in 3 ml Acetonitril wurde die Lösung äquivalenter Mengen von 2-Chlor-4,5-difluor-benzoyl-isocyanat getropft und die Mischung 30 Minuten bei Raumtemperatur gerührt. Der Feststoff wurde dann abgesaugt, mit Isopropanol verrührt und im Vakuum getrocknet.
Ausbeute: 50 mg Fp.: 227,5 °C

### Beispiel 4:

### a) [5-(3-Methoxy-4-nitro-phenyl)-4H-[1,2,4]triazol-3-yl]-essigsäureethylester

Zur Lösung von 633 mg 3-Methoxy-4-nitro-benzoesäurehydrazid in 2 ml N-Methylpyrrolidon wurden 477 mg Malonsäure-ethylester-imidsäure-ethylester Hydrochlorid gegeben und die Mischung 3 Stunden auf 140 °C erhitzt. Nach dem Erkalten wurde die Mischung mit 50 ml Wasser versetzt und das Produkt mit Essigsäureethylester ausgeschüttelt. Nach dem Trocknen und Einengen der organischen Phase wurde säulenchromatografisch (Kieselgel, LM: Dichlormethan:Methanol = 95:5) gereinigt.
Ausbeute: 220 mg Fp.: Öl

### b) [5-(4-Amino-3-methoxy-phenyl)-4H-[1,2,4]triazol-3-yl]-essigsäureethylester

Zur Lösung von 200 mg [5-(3-Methoxy-4-nitro-phenyl)-4H-[1,2,4]triazol-3-yl]-essigsäureethylester in 100 ml Ethanol wurden 50 mg Pd/C gegeben und in die Mischung bei Raumtemperatur Wasserstoff eingeleitet, bis die theoretische Menge aufgenommen war. Dann wurde der Katalysator durch Filtration abgetrennt und das Filtrat eingeengt.
Ausbeute: 140 mg Fp.: Öl

### c) [5-(3-Methoxy-4-nitro-phenyl)-4H-[1,2,4]triazol-3-yl]-essigsäure

Die Mischung von 140 mg [5-(3-Methoxy-4-nitro-phenyl)-4H-[1,2,4]triazol-3-yl]-essigsäureethylester, 5 ml Methanol und 1 ml 1 N Natronlauge wurde 2 Stunden bei Raumtemperatur gerührt. Danach wurden die flüchtigen Anteile am Rotationsverdampfer abgezogen, der Rückstand mit 10 ml Wasser verdünnt und mit 1 N Salzsäure auf pH 5 gestellt.Nach dem Verrühren wurde der Feststoff abgesaugt.
Ausbeute: 99 mg Fp.: 135,5 °C

### d) (5-(4-(3-(2-Chlor-4-fluor-benzoyl)-ureido)-3-methoxy-phenyl)-4H-[1,2,4]triazol-3-yl)-essigsäure

Zur Lösung von 89 mg [5-(3-Methoxy-4-nitro-phenyl)-4H-[1,2,4]triazol-3-yl]-essigsäureethylester in 5 ml Acetonitril wurde die Lösung äquivalenter Mengen von 2-Chlor-4-fluor-benzoyl-isocyanat in Acetonitril getropft und die Mischung über Nacht bei Raumtemperatur gerührt. Der gebildete Feststoff wurde dann abgesaugt und im Vakuum getrocknet.
Ausbeute: 70 mg Fp.: >300 °C (Zers.)

### Beispiel 5:

### a) 2-[3-Methyl-5-(2-nitro-phenyl)-[1,2,4]triazol-4-yl]-benzoesäure

Die Mischung bestehend aus 1,8 g 2-Nitro-benzoesäurehydrazid, 1,6 g 2-Methyl-benzo[d][1,3]oxazin-4-on und 5 ml N-Methylpyrrolidon wurde 2 Stunden auf 80 °C unter Umrühren erhitzt. Nach dem Erkalten wurde mit Wasser bis zur leichten Trübung versetzt und 1 Stunde lang nachgerührt, wobei ein Niederschlag ausfiel, der abgesaugt, aus Ethanol umkristallisiert und im Vakuum getrocknet wurde.
Ausbeute: 0,89 g Fp.: 228,7 °C

### b) 2-[3-Methyl-5-(4-nitro-phenyl)-[1,2,4]triazol-4-yl]-benzoesäure

wurde entsprechend aus 4-Nitro-benzoesäurehydrazid hergestellt und aus Isopropanol umkristallisiert.
Ausbeute: 0,6 g Fp.: 275,4 °C

### c) 2-[3-(2-Amino-phenyl)-5-methyl-[1,2,4]triazol-4-yl]-benzoesäure

wurde durch Hydrierung von 400 mg 2-[3-Methyl-5-(2-nitro-phenyl)-[1,2,4]triazol-4-yl]-benzoesäure in Gegenwart von Pd/C in Tetrahydrofuran hergestellt und durch Verrühren mit Dichlormethan gereinigt.
Ausbeute: 240 mg Fp.: 179,4 °C

### d) 2-[3-(4-Amino-phenyl)-5-methyl-[1,2,4]triazol-4-yl]-benzoesäure

wurde durch Hydrierung von 270 mg 2-[3-Methyl-5-(4-nitro-phenyl)-[1,2,4]triazol-4-yl]-benzoesäure in Gegenwart von Pd/C in Tetrahydrofuran hergestellt und durch Säulenchromatografie (Kieselgel, LM: Dichlormethan:Methanol = 95:5) gereinigt.
Ausbeute: 75 mg Fp.: 207,8 °C

### e) 2-(3-{2-[3-(2-Chlor-4,5-difluor-benzoyl)-ureido]-phenyl}-5-methyl-[1,2,4]triazol-4-yl)-benzoesäure

Zur Lösung von 88 mg 2-[3-(2-Amino-phenyl)-5-methyl-[1,2,4]triazol-4-yl]-benzoesäure in 3 ml Acetonitril wurde die Lösung äquivalenter Mengen von 2-Chlor-4-fluor-benzoyl-isocyanat getropft und die Mischung 30 Minuten bei Raumtemperatur gerührt. Der Feststoff wurde abgesaugt und im Vakuum getrocknet.
Ausbeute: 120 mg Fp.: 194,7 °C

### Beispiel 6:

### a) 4-Chlor-3-nitro-benzoesäure-amidrazon Hydrochlorid

Die Mischung bestehend aus 6,8 g 4-Chlor-3-nitro-benzimidsäureethylester Hydrochlorid, 100 ml Isopropanol und 3,75 ml Hydrazinhydrat wurde 60 Minuten bei Raumtemperatur gerührt. Der Niederschlag wurde abgesaugt und mit 50 ml Isopropanol kurz verrührt, abgeaugt und im Vakuum getrocknet.
Ausbeute: 3,95 g Fp.: 150,2 °C

### b) 3-(4-Chlor-3-nitro-phenyl)-5-methyl-4H-[1,2,4] triazol

Die Mischung bestehend aus 322 mg 4-Chlor-3-nitro-benzoesäure-amidrazon Hydrochlorid, 12 ml Toluol und 0,21 ml Orthoessigsäuretrimethylester wurde 60 Minuten auf 110 °C erhitzt. Das Lösemittel wurde im Vakuum bei 40 °C abgezogen und der Rückstand säulenchromatografisch (Kieselgel; Dichlormethan:Methanol = 98:2) gereinigt.
Ausbeute: 65 mg Fp.: 167,9 °C

### c) 3-(3-Amino-4-chlor-phenyl)-5-methyl-4H-[1,2,4]-triazol

Die Mischung bestehend aus 120 mg 3-(4-Chlor-3-nitro-phenyl)-5-methyl-4H-[1,2,4]triazol, 30 ml Essigester und 644 mg Zinnchlorid wurde 6 Stunden am Rückfluss erhitzt. Nach dem Erkalten wurde mit Wasser gewaschen, die organische Phase über Natriumsulfat getrocknet und im Vakuum bei 40 °C eingeengt.
Ausbeute: 90 mg Fp.: Harz

### d) 1-(2-Chlor-4,5-difluor-benzoyl)-3-[2-chlor-5-(5-methyl-4H-[1,2,4]triazol-3-yl)-phenyl]-harnstoff

Zur Lösung von 85 mg 3-(3-Amino-4-chlor-phenyl)-5-methyl-4H-[1,2,4]-triazol in 8 ml Acetonitril wurde die Lösung äquivalenter Mengen von 2-Chlor-4,5-difluor-benzoyl-isocyanat getropft und die Mischung 2 Stunden bei Raumtemperatur gerührt. Der gebildete Feststoff wurde dann abgesaugt und im Vakuum getrocknet.
Ausbeute: 65 mg Fp.: 227,4 °C

### Analog wurden hergestellt:

### e) 1-(2-Chlor-4,5-difluor-benzoyl)-3-[2-chlor-5-(4H-[1,2,4]triazol-3-yl)-phenyl]-harnstoff Fp.: 294,5 °C

### f) 1-(2-Chlor-4,5-difluor-benzoyl)-3-[2-trifluormethoxy-4-(5-hydroxy-1H-[1,2,4]triazol-3-yl)-phenyl]-harnstoff

Fp.: >350 °C

### Beispiel 7:

### a) 2-Chlor-4-(1H-tetrazol-5-yl)-phenylamin

Die Mischung bestehend aus 1,07 g 4-Amino-3-chlorbenzonitril , 30 ml Xylol und 1,7 g Trimethylzinnazid wurde 8 Stunden bei 135 °C gerührt. Nach dem Erkalten wurden 25 ml Methanol zugefügt, 30 Minuten bei Raumtemperatur gerührt und die leichtflüchtigen Anteile am Rotationsverdampfer abgezogen. Aus der so erhaltenen Mischung fiel beim Verrühren ein Feststoff aus, der abgesaugt und kurz im Vakuum getrocknet wurde. Dieser Feststoff wurde in 1 N Natronlauge gelöst, filtriert und das Produkt durch ansäuern mit 2N Salzsäure gefällt, abgesaugt und im Vakuum getrocknet.
Ausbeute: 1,24 g Fp.: 183,8 °C

### b) 4-(1H-Tetrazol-5-yl)-2-trifluoromethoxy-phenylamin

wurde analog aus 505 mg 4-Amino-3-trifluormethoxy-benzonitril erhalten.
Ausbeute: 360 mg Fp.: 183,0 °C

### c) 1-(2-Chlor-4-fluor-benzoyl)-3-[2-chlor-4-(1H-tetrazol-5-yl)-phenyl]-harnstoff

Zur Lösung von 100 mg 2-Chlor-4-(1H-tetrazol-5-yl)-phenylamin in 3 ml Acetonitril wurde die Lösung äquivalenter Mengen von 2-Chlor-4-fluor-benzoyl-isocyanat getropft und die Mischung 60 Minuten bei 40 °C gerührt. Der gebildete Feststoff wurde abgesaugt und im Vakuum getrocknet.
Ausbeute: 115 mg Fp.: 227,6 °C

### d) 1-(2-Chlor-4,5-difluor-benzoyl)-3-[2-chlor-4-(1H-tetrazol-5-yl)-phenyl]-harnstoff

zur Lösung von 100 mg 2-chlor-4-(1H-tetrazol-5-yl)-phenylamin in 3 ml Acetonitril wurde die Lösung äquivalenter Mengen von 2-Chlor-4,5-difluor-benzoyl-isocyanat getropft und die Mischung 60 Minuten bei 40 °C gerührt. Der gebildete Feststoff wurde abgesaugt und im Vakuum getrocknet.
Ausbeute: 86 mg Fp.: >300 °C

### e) 1-(2-Chlor-4-fluor-benzoyl)-3-[4-(1H-tetrazol-5-yl)-2-trifluoromethoxy-phenyl]-harnstoff

wurde analog aus 100 mg 5-(4-flmino-3-trifluormethoxy-phenyl)-tetrazol erhalten. Ausbeute: 56 mg Fp.: 276 °C

### f) 1-(2-Chlor-4,5-difluor-benzoyl)-3-[4-(1H-tetrazol-5-yl)-2-trifluormethoxy-phenyl]-harnstoff

wurde analog aus 100 mg 4-(1H-Tetrazol-5-yl)-2-trifluormethoxy-phenylamin und 2-Chlor-4,5-difluor-benzoyl-isocyanat erhalten.
Ausbeute: 98 mg Fp.: 215,0 °C

### Beispiel 8:

### a) 5-(3-Methoxy-4-nitro-phenyl)-3H-[1,3,4]oxdiazol-2-on

Zur Lösung von 850 mg 3-Methoxy-4-nitro-benzoesäurehydrazid (Fp.: 158,2 °C, aus 3-Methoxy-4-nitro-benzoesäuremethylester und Hydrazinhydrat in Isopropanol bei 80 °C hergestellt) in 25 ml Dioxan wurden 1,2 Äquivalente einer 20 %igen Phosgenlösung in Toluol getropft und die Mischung 1 Stunde bei Raumtemperatur gerührt. Nach dem Einengen wurde der Rückstand aus Isopropanol umkristallisiert abgesaugt und im Vakuum getrocknet.
Ausbeute: 620 mg Fp.: 223,1 °C

### b) 5-(4-Amino-3-methoxy-phenyl)-3H-[1,3,4]oxdiazol-2-on

In die Mischung von 550 mg 5-(3-Methoxy-4-nitro-phenyl)-3H-[1,3,4]oxdiazol-2-on, 100 mg Pd/C und 50 ml Tetrahydrofuran wurde Wasserstoff unter Normaldruck bis zur theoretischen Aufnahme eingeleitet. Danach wurde vom Katalysator abgesaugt und die Mischung im Vakuum zur Trockne eingeengt.
Ausbeute: 500 mg Fp.: 206,3 °C

### c) 1-(2-Chlor-4-fluor-benzoyl)-3-[4-(5-hydroxy-[1,3,4]oxdiazol-2-yl)-2-methoxy-phenyl]-harnstoff

Zur Lösung von 103 mg 25-(4-Amino-3-methoxy-phenyl)-3H-[1,3,4]oxdiazol-2-on in 5 ml Acetonitril wurde die Lösung äquivalenter Mengen von 2-Chlor-4-fluor-benzoyl-isocyanat in Acetonitril getropft und die Mischung über Nacht bei Raumtemperatur gerührt. Der gebildete Feststoff wurde abgesaugt und im Vakuum getrocknet. Ausbeute: 155 mg Fp.: 280,7 °C

Analog wurden hergestellt:
d) 1-(2-Chlor-4-fluor-benzoyl)-3-[2-(5-hydroxy-[1,3,4]oxdiazol-2-yl)-phenyl]-harnstoff Fp.: 229,7 °C
e) 1-(2-Chlor-4,5-difluor-benzoyl)-3-[4-(5-hydroxy-[1,3,4]oxdiazol-2-yl)-2-methoxyphenyl]-harnstoff
   Fp.: 293,1 °C
f) 1-(2-Chlor-4,5-difluor-benzoyl)-3-[2-(5-hydroxy-[1,3,4]oxdiazol-2-yl)-phenyl]-harnstoff Fp.: 222,9 °C
g) 1-(2-Chlor-4-fluor-benzoyl)-3-(2-[1,3,4]oxdiazol-2-yl-phenyl)-harnstoff
   Fp. : 204,0 °C
h) 1-(2-Chlor-4,5-difluor-benzoyl)-3-(2-[1,3,4]oxdiazol-2-yl-phenyl)-harnstoff
   Fp. : 199,6 °C

### Beispiel 9:

### a) 3-Methoxy-4-nitro-phenylhydrazin

Zur Lösung von 3,2 g 4-Fluor-2-methoxy-nitrobenzol in 15 ml N-Methylpyrrolidon wurden 4,5 ml Hydrazinhydrat getropft und die Mischung 2 Stunden gerührt, wobei anfangs leichte Erwärmung auftrat. Darauf wurde die Mischung mit 50 ml Wasser verdünnt und verrührt, wobei sich ein Niederschlag bildete, der abgesaugt und im Vakuum getrocknet wurde.
Ausbeute: 3,25 g Fp.: 162,5 °C

### b) N'-(3-Methoxy-4-nitro-phenyl)-hydrazinoameisensäure-methylester

Zur Lösung von 3,0 g 3-Methoxy-4-nitro-phenylhydrazin in 25 ml Dichlormethan und 6,6 ml Pyridin wurden bei Raumtemperatur langsam 1,5 ml Chlorameisensäuremethylester zugetropft. Nach 2 Stunden wurden die flüchtigen Anteile am Rotationsverdampfer im Vakuum entfernt, der Rückstand in Wasser aufgenommen und nach dem schwachen Ansäuern mit 2N Salzsäure mit Essigsäureethylester extrahiert. Nach dem Trocknen und Einengen der Essigsäureethylesterphase verblieb ein fester Rückstand, der aus Isopropanol umkristallisiert wurde.
Ausbeute: 3,07 g Fp.: 143,7 °C

### c) 5-Methoxy-3-(3-methoxy-4-nitro-phenyl)-3H-[1,3,4]oxdiazol-2-on

Die Mischung bestehend aus 3,05 g N'-(3-Methoxy-4-nitro-phenyl)-hydrazinoameisensäure-methylester, 30 ml Dichlormethan, 5,2 ml Pyridin und 16,5 ml einer 20%igen toluolischen Phosgenlösung wurde 1 Stunde bei Raumtemperatur gerührt. Nach dem Einengen im Vakuum wurde der halbfeste Rückstand mit 50 ml Wasser unter Zugabe von 3 ml 2N Salzsäure verrührt, der Feststoff abgesaugt und im Vakuum bei Raumtemperatur getrocknet.
Ausbeute: 2,8 g Fp.: 145,1 °C

### d) 3-(4-Amino-3-methoxy-phenyl)-5-methoxy-3H-[1,3,4]oxdiazol-2-on Hydrochlorid

In die Mischung bestehend aus 2,8 g 5-Methoxy-3-(3-methoxy-4-nitro-pheriyl)-3H-[1,3,4]oxdiazol-2-on, 250 ml Methanol und 0,3 g Pd/C wurde bei Raumtemperatur Wasserstoff eingeleitet, bis die theoretische Menge aufgenommen war. Dann wurde der Katalysator durch Filtration abgetrennt und das Filtrat eingeengt. Der Rückstand wurde in Essigsäureethylester aufgenommen, das Produkt mit methanolischer Salzsäure gefällt, abgesaugt und im Vakuum getrocknet.
Ausbeute: 2,0 g Fp.: 245,9 °C

### e) 1-(2'-Chlor-4-fluor-benzoyl)-3-(2-methoxy-4-(5-methoxy-2-oxo-3H-[1,3,4]-oxdiazol-3-yl)-phenyl)-harnstoff

Zur Lösung von 0,39 g 5-Methoxy-3-(4-amino-3-methoxy-phenyl)-3H-[1,3,4]oxdiazol-2-on Hydrochlorid und 0,2 ml Triethylamin in 5 ml Acetonitril wurde die Lösung äquivalenter Mengen von 2-Chlor-4-fluor-benzoyl-isocyanat in Acetonitril getropft und die Mischung über Nacht bei Raumtemperatur gerührt. Der gebildete Feststoff wurde abgesaugt und im Vakuum getrocknet.
Ausbeute: 0,16 g Fp.: 211,1 °C

### Analog wurde hergestellt:

### f) 1-(2-Chlor-4-fluor-benzoyl)-3-(2-methyl-4-(5-methylamino-2-oxo-3H-[1,3,4]-oxdiazol-3-yl)-phenyl)-harnstoff

Fp.: 198,0 °C

### g) 1-(2-Chlor-4-fluor-benzoyl)3-[2-chlor-4-(5-methyl-[1,3,4]oxadiazol-2-yl)-phenyl]-harnstoff

Die Mischung von 138 mg 1-(2-Chlor-4-fluor-benzoyl)-3-[2-chlor-4-(1H-tetrazol-5-yl)-phenyl]-harnstoff, 180 mg Acetanhydrid, 260 mg Pyridin und 3 ml Dioxan wurde 8 Stunden auf 80 °C erhitzt. Nach dem Einrotieren im Vakuum wurde der Rückstand mit Wasser/Eisessig verrührt, der gebildete Feststoff abgesaugt, in Dichlormethan/Methanol (1:1) gelöst und die Lösung durch Filtration vom unlöslichen Anteil getrennt. Nach dem Einengen im Vakuum wurde der Rückstand mit Ethanol verrührt und der Feststoff abgesaugt.
Ausbeute: 11 mg Fp.: 198,2 °C

### Beispiel 10:

### a) 5-(2-Nitro-phenyl)-[1,3,4]oxdiazol-2-yl-amin

Zur Lösung von 3,6 g 2-Nitro-benzoesäure-hydrazid in 20 ml Acetonitril wurden 4 ml einer 5M Bromcyanlösung in Acetonitril getropft. Dabei trat erst eine klare Lösung auf, dann fiel ein Niederschlag aus, der nach kurzem Nachrühren abgesaugt, mit Acetonitril nachgewaschen und im Vakuum getrocknet wurde.
Ausbeute: 4,3 g Fp.: 211,2 °C

### b) 5-(2-Amino-phenyl)-[1,3,4]oxdiazol-2-yl-amin

In die Lösung von 350 mg 5-(2-Nitro-phenyl)-[1,3,4]oxdiazol-2-yl-amin wurde unter Normaldruck Wasserstoff eingeleitet, bis die theoretische Menge aufgenommen war. Nach dem Absaugen des Katalysators und Einengen des Gemisches am Vakuum wurde der verbleibende ölige Rückstand durch Säulenchromatografie gereinigt (LM: Dichlormethan:Methanol = 95:5; Kieselgel).
Ausbeute: 200 mg Fp.: 199,0 °C

### c) 1-[2-(5-Amino-[1,3,4]oxdiazol-2-yl)-phenyl]-3-(2-chlor-4,5-difluor-benzoyl)-harnstoff

Zur Lösung von 70 mg 5-(2-Amino-phenyl)-[1,3,4]oxdiazol-2-yl-amin in 2 ml Acetonitril wurde die Lösung äquivalenter Mengen von 2-Chlor-4-fluor-benzoyl-isocyanat getropft und die Mischung 30 Minuten bei Raumtemperatur gerührt. Der Feststoff wurde dann abgesaugt und im Vakuum getrocknet.
Ausbeute: 95 mg Fp.: 205,8 °C

### Beispiel 11:

### a) N-[4-(N-Hydroxycarbamimidoyl)-2-trifluormethoxy-phenyl]-acetamid

Die Mischung bestehend aus 610 mg N-(4-Cyano-2-trifluormethoxy-phenyl)-acetamid, 15 ml Isopropanol, 255 mg Hydroxylamin Hydrochlorid und 410 mg Natriumacetat wurde 5 Stunden unter Rückfluss gekocht. Nach dem Erkalten wurde der unlösliche Anteil abfiltriert, das Filtrat eingeengt, in wenig Isopropanol aufgenommen, das Produkt durch Zugabe von Wasser bis zur ersten Trübung und anschließendem Verrühren ausgefällt, abgesaugt und getrocknet.
Ausbeute: 280 mg Fp.: 178,2 °C

### b) N-[4-(5-Oxo-4,5-dihydro-[1,2,4]oxadiazol-3-yl)-2-trifluormethoxy-phenyl]-acetamide

Die Mischung bestehend aus 130 mg N-[4-(N-Hydroxycarbamimidoyl)-2-trifluormethoxy-phenyl]-acetamid, 2 ml N-Methylpyrrolidon, 0,55 ml Pyridin und 0,049 ml Chlorameisensäure-ethylester wurde 5 Stunden bei 80 °C gerührt. Nach dem Erkalten wurde mit Wasser verdünnt und das Produkt mit 20 ml Essigsäureethylester ausgeschüttelt. Die organische Phase wurde über Natriumsulfat getrocknet und im Vakuum bei 40 °C eingedampft.
Ausbeute: 200 mg Fp.: Harz

### c) 3-(4-Amino-3-trifluormethoxy-phenyl)-4H-[1,2,4]-oxdiazol-5-on Hydrochlorid

Die Mischung bestehend aus 200 mg N-[4-(5-Oxo-4-,5-dihydro-[1,2,4]oxadiazol-3-yl)-2-trifluormethoxy-phenyl]-acetamid, 10 ml Methanol und 0,5 ml 4M Lösung von Salzsäure in Dioxan wurde 15 Stunden bei Raumtemperatur gerührt. Nach dem Einrotieren der flüchtige Anteile verblieb ein gelbliches Öl.
Ausbeute: 190 mg Fp.: Öl

### d) 1-(2-Chlor-4,5-difluor-benzoyl)-3-[4-(5-oxo-4,5-dihydro-[1,2,4]oxdiazol-3-yl)-2-trifluormethoxy-phenyl]-harnstoff

Zur Lösung von 95 mg 3-(4-Amino-3-trifluorrnethoxy-phenyl)- 4H-[1,2,4]-oxdiazol-5-on Hydrochlorid und 0,054 ml Hünigbase in 4 ml Acetonitril wurde die Lösung äquivalenter Mengen von 2-Chlor-4,5-difluor-benzoyl-isocyanat getropft und die Mischung 30 Minuten bei Raumtemperatur gerührt. Der Feststoff wurde dann abgesaugt und im Vakuum getrocknet.
Ausbeute: 55 mg Fp.: 224,5 °C

Analog wurden die folgenden Beispiele hergestellt:

### e) 1-(2-Chlor-4-fluor-benzoyl)-3-[4-(5-oxo-4,5-dihydro-[1,2,4]oxdiazol-3-yl)-2-trifluormethoxy-phenyl]-harnstoff

Fp. 230,1 °C

### f) 1-(2-Chlor-4-fluor-benzoyl)-3-[2-chlor-4-(5-oxo-4,5-dihydro-[1,2,4]oxdiazol-3-yl)-phenyl]-harnstoff

Fp. 243,6 °C

### Beispiel 12:

### a) 5-Methyl-2-(3-methyl-4-nitro-phenyl)-1,2-dihydro-pyrazol-3-on

Die Mischung von 500 mg 3-Methyl-4-nitro-phenylhydrazin, 0,32 ml Acetessigsäuremethylester und 20 ml Toluol wurde 8 Stunden auf 100°C erhitzt. Nach dem Erkalten wurde das Reaktionsgemisch am Vakuum eingeengt und der Rückstand in t-Butyl-methylether verrührt. Der Feststoff wurde abgesaugt und im Vakuum getrocknet.
Ausbeute: 380 mg Fp.: 179,0 °C

### b) 2-(4-Amino-3-methyl-phenyl)-5-methyl-1,2-dihydro-pyrazol-3-on

In die Mischung von 350 mg 5-Methyl-2-(3-methyl-4-nitro-phenyl)-1,2-dihydro-pyrazol-3-on, 70 mg Pd/C und 50 ml Tetrahydrofuran wurde Wasserstoff unter Normaldruck bis zur theoretischen Aufnahme eingeleitet. Danach wurde vom Katalysator abgesaugt, die Mischung im Vakuum zur Trockne eingeengt und der Rückstand in t-Butyl-methylether verrührt. Der Feststoff wurde abgesaugt und im Vakuum getrocknet. Ausbeute: 280 mg Fp.: 59,3 °C

### c) 1-(2-Chlor-4-fluor-benzoyl)-3-[2-methyl-4-(3-methyl-5-oxo-2,5-dihydro-pyrazol-1-yl)-phenyl]-harnstoff

Zur Lösung von 71 mg 2-(4-Amino-3-methyl-phenyl)-5-methyl-1,2-dihydro-pyrazol-3-on in 6 ml Acetonitril wurde die Lösung äquivalenter Mengen von 2-Chlor-4-fluor-benzoyl-isocyanat in Acetonitril getropft und die Mischung über Nacht bei Raumtemperatur gerührt. Der gebildete Feststoff wurde abgesaugt und im Vakuum getrocknet.
Ausbeute: 70 mg Fp.: 225,5 °C

### Beispiel 13:

### a) 1-[2-(1H-Benzoimidazol-2-yl)-phenyl]-3-(2-chlor-4,5-difluor-benzoyl)-harnstoff

Zur Lösung von 142 mg 2-(1H-Benzimidazol-2-yl)-phenylamin in 8 ml Acetonitril wurde die Lösung äquivalenter Mengen von 2-Chlor-4,5-difluor-benzoyl-isocyanat getropft und die Mischung 30 Minuten bei Raumtemperatur gerührt. Der Feststoff wurde dann abgesaugt und im Vakuum getrocknet.
Ausbeute: 250 mg Fp.: 268 °C (Zers.)

### Analog wurden hergestellt:

### b) 5-{2-[3-(2-Chlor-4,5-difluor-benzoyl)-ureido]-phenyl}-furan-2-carbonsäure

Fp. 239,3 °C

### c) 5-{2-[3-(2-Chlor-4-fluor-benzoyl)-ureido]-phenyl}-furan-2-carbonsäure

Fp. 236,3 °C

### Beispiel 14:

### a) 3-(4-Chlor-3-nitro-phenyl)-6-methyl-4H-[1,2,4]triazin-5-on

Die Mischung bestehend aus 322 mg 4-Chlor-3-nitro-benzoesäure-amidrazon Hydrochlorid, 12 ml Ethanol und 0,18 ml Ethylpyruvat wurde 60 Minuten auf 80 °C erhitzt. Nach dem Erkalten wurde der Niederschlag abgesaugt, mit etwas Ethanol gewaschen und im Vakuum bei 40 °C getrocknet.
Ausbeute: 115 mg Fp.: 247,1 °C

### b) 3-(4-Chlor-3-nitro-phenyl)-5,6-dimethyl-[1,2,4]triazine

Diese Verbindung wurde analog dem vorstehenden Beispiel ausgehend von 2,3-Butandion erhalten.
Fp.: 167,7 °C

### c) 3-(3-Amino-4-chlor-phenyl)-6-methyl-4H-[1,2,4]triazin-5-on

Diese Verbindung wurde durch Reduktion von 3-(4-Chlor-3-nitro-phenyl)-6-methyl-4H-[1,2,4]triazin-5-on mit Zinnchlorid erhalten.
Fp.: 258,1 °C

### d) 2-Chlor-5-(5,6-dimethyl-[1,2,4]triazin-3-yl)-phenylamin

Diese Verbindung wurde durch Reduktion von 3-(4-Chlor-3-nitro-phenyl)-5,6-dimethyl-[1,2,4]triazine mit Zinnchlorid erhalten.
Fp.: 211,8 °C

### e) 1-(2-Chlor-4,5-difluor-benzoyl)-3-[2-chlor-5-(6-methyl-5-oxo-4,5-dihydro-[1,2,4]triazin-3-yl)-phenyl]-harnstoff

Zur Lösung von 60 mg 3-(3-Amino-4-chlor-phenyl)-6-methyl-4H-[1,2,4]triazin-5-on in 8 ml Acetonitril wurde die Lösung äquivalenter Mengen von 2-Chlor-4,5-difluor-benzoyl-isocyanat getropft und die Mischung 30 Minuten bei Raumtemperatur gerührt. Der Feststoff wurde abgesaugt und im Vakuum getrocknet.
Ausbeute: 75 mg Fp.: 236,6 °C

### f) 1-(2-Chlor-4,5-difluor-benzoyl)-3-[2-chlor-5-(5,6-dimethyl-[1,2,4]triazin-3-yl)-phenyl]-harnstoff

Zur Lösung von 75 mg 2-Chlor-5-(5,6-dimethyl-[1,2,4]triazin-3-yl)-phenylamin in 8 ml Acetonitril wurde die Lösung äquivalenter Mengen von 2-Chlor-4,5-difluor-benzoyl-isocyanat getropft und die Mischung 30 Minuten bei Raumtemperatur gerührt. Der Feststoff wurde abgesaugt und im Vakuum getrocknet.
Ausbeute: 105 mg Fp.: 229,2 °C

### Beispiel 15:

### a) 1-(4-Fluor-2-nitro-phenyl)-piperidin-3-carbonsäureamid

Die Mischung bestehend aus 1,62 g 2,5-Difluor-nitrobenzol, 1,9 g Nipecotamid und 10 ml NMP wurde 2 Stunden auf 80°C unter Umrühren erhitzt. Nach dem Erkaltenlassen wurden 30 ml Wasser zugefügt und die Mischung 30 Minuten lang bei RT gerührt. Der ausgefallene Niederschlag wurde abgesaugt und im Vakuum getrocknet.
Ausbeute: 2,8 g Fp.: 142,5°C

### b) 1-(2-Amino-4-fluor-phenyl)-piperidin-3-carbonsäureamid-hydrochlorid

Die Lösung von 2,67 g 1-(4-Fluor-2-nitro-phenyl)-piperidin-3-carbonsäureamid in 100 ml THF wurde mit 260 mg Pd/C versetzt. Diese Mischung wurde unter Normaldruck in der Schüttelente hydriert, bis die theoretische Wasserstoffaufnahme erfolgt ist. Dann wird die Mischung mit in Essigester gelöstem Chlorwasserstoff angesäuert, der Feststoff abgesaugt und mit Methanol gewaschen und das Filtrat im Vakuum eingeeengt. Der Rückstand wird mit Tert. Butylmethylether verrieben und der Feststoff abgesaugt und im Vakuum getrocknet.
Ausbeute: 2,45 g Fp. 159,2°C

### c) 1-{2-[3-(2-Chlor-4,5-difluor-benzoyl)-ureido]-4-fluor-phenyl}-piperidin-3-carbonamide

In die Lösung von 109 mg 1-(2-Amino-4-fluor-phenyl)-piperidin-3-carbonsäureamid-hydrochlorid in 5 ml Acetonitril wurde unter Umrühren die äquimolare Lösung von 2-Chlor-4,5-difluor-benzoylisocyanat in Acetonitril getropft. Die Mischung wurde 6 bei RT gerührt und der Niederschlag abgesaugt und im Vakuum bei RT getrocknet.
Ausbeute: 150 mg Fp.: 216,0°C

### Beispiel 16:

### a) 1-(4-Fluor-2-nitro-phenyl)-piperidin-4-carbonsäure

Die Mischung bestehend aus 1,62 g 2,5-Difluor-nitrobenzol, 1,9 g Piperidin-4-carbonsäure und 10 ml NMP wurde 2 Stunden auf 80°C unter Umrühren erhitzt. Nach dem Erkaltenlassen wurden 30 ml Wasser zugefügt und die Mischung mit 2N-Salzsäure schwach sauer gestellt und bei RT verrührt. Der ausgefallene Niederschlag wurde abgesaugt und im Vakuum getrocknet.
Ausbeute: 3,4 g Fp.: 143,7°C

### b) 1-(2-Amino-4-Fluor-phenyl)-piperidin-4-carbonsäure-hydrochlorid

Die Lösung von 804 mg 1-(4-Fluor-2-nitro-phenyl)-piperidin-4-carbonsäure in 40 ml Essigester wurde mit 3,8 g Zinn-2-Chlorid versetzt und 2 Stunden bei RT gerührt. Dann wurden 50 ml Wasser zugesetzt und die Mischung über eine Klärschicht filtriert. Die Essigesterphase wurde abgetrennt, über Natriumsulfat getrocknet und einrotiert, wobei ein halbfester Rückstand verblieb, der direkt der weiteren Umsetzung unterworfen wurde.
Ausbeute: 395 mg Fp.: Rohprodukt

### c) 1-{2-[3-(2-Chlor-4,5-difluor-benzoyl)-ureido]-4-fluor-phenyl}-piperidin-4-carbonsäure

In die Lösung von 53 mg 1-(2-Amino-4-fluor-phenyl)-piperidin-4-carbonsäurehydrochlorid in 3 ml Acetonitril wurde unter Umrühren die äquimolare Lösung von 2-Chlor-4,5-difluor-benzoylisocyanat in Acetonitril getropft. Die Mischung wurde 6 bei RT gerührt und der Niederschlag abgesaugt und im Vakuum bei RT getrocknet.
Ausbeute: 75 mg Fp.: 215,9°C

### Beispiel 39:

### c) 1-{2-[3-(2-Chlor-4,5-difluor-benzoyl)-ureido]-4-fluor-phenyl}-piperidin-4-carbonsäure- Natriumsalz

Die 60°C warme Lösung von 100 mg 1-{2-[3-(2-Chlor-4,5-difluor-benzoyl)-ureido]-4-fluor-phenyl}-piperidin-4- carbonsäure in 8 ml Isopropanol wird mit der äquimolaren Menge 2N Natronlauge versetzt und nach Zugabe von 20 ml Wasser unter Umrühren langsam abgekühlt. Das ausgefallene Produkt wird abgesaugt, mit Isopropanol und Wasser gewaschen und im Vakuum getrocknet.
Ausbeute: 85 mg Fp. 160°C (Zers.)

### Beispiel 41:

### c) 1-{2-[3-(2-Chlor-4,5-difluor-benzoyl)-ureido]-4-chlor-phenyl}-piperidin-4-carbonsäure

Die Mischung bestehend aus 90 mg 1-{2-[3-(2-Chlor-4,5-difluor-benzoyl)-ureido]-4-chlor-phenyl}-piperidin-4- carbonsäuremethylester (Bspl.15), 9,6 mg Lithiumhydroxid, 3 ml Wasser, 3 ml Methanol und 3 ml THF wird 36 Stunden bei Raumtemperatur stehen gelassen. Die flüchtigen Anteile werden im Vakuum bei RT abgezogen und der Rest mit 2N Salzsäure auf pH = 4 gestellt. Der sich dabei bildende Niederschlag wird abgesaugt und säulenchromatografisch gereinigt (Kieselgel, LM: Methylenchlorid: Methanol = 9:1).
Ausbeute: 30 mg Fp.: Harz

Die Verbindungen der Formel I können hergestellt werden, dadurch dass Harnstoffe der Formel 2 oder Anilinderivate der Formel 3 mit Aroyl-isocyanaten, mit reaktiven Säurederivaten, mit Säurechloriden oder mit Anhydriden, der Formel 4, 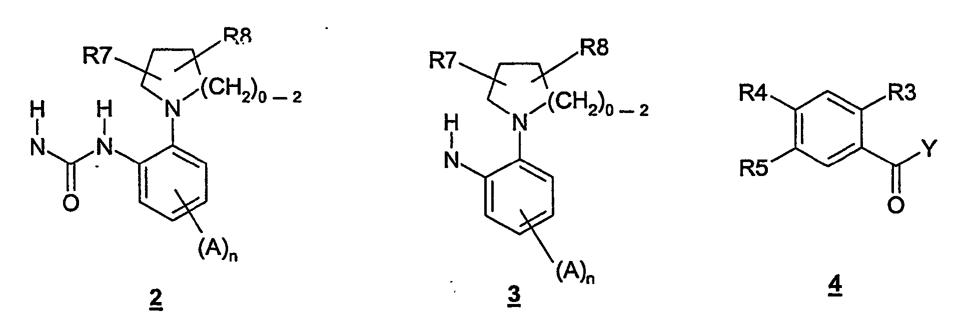 worin R3, R4, R5, R7, R8, A und X die oben angegebenen Bedeutungen haben, umgesetzt werden.

**Tabelle 1**

| | | | | | | | |
|---|---|---|---|---|---|---|---|
| Verbindungen der Formel l | | | | | | | |
| 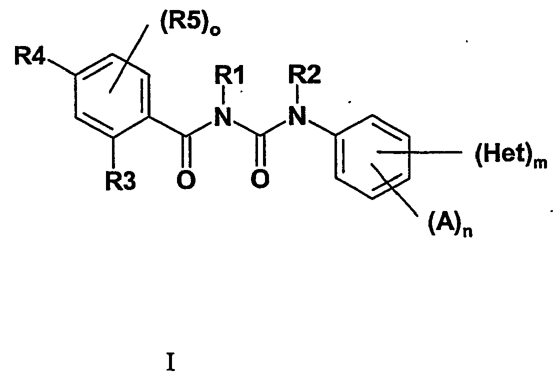 | | | | | | | |

| **Bsp.** | **R1** | **R2** | **R3** | **R4** | **(R5)ₒ** | **(A)ₙ** | **(Het)ₘ** |
|---|---|---|---|---|---|---|---|
| | | | | | | | |
| 1c | H | H | Cl | F | 5-F | 2-Fluor | 4-((1,2,4)-Triazol-1-yl) |
| 2c | H | H | Cl | F | H | 2-OMe | 4-(5-Methyl-4H-(1,2,4)-triazol-3-yl) |
| 3 | H | H | Cl | F | 5-F | 2-OMe | 4-(5-Methyl-4H-(1,2,4)-triazol-3-yl) |
| 4d | H | H | Cl | F | H | 2-OMe | 4-(1,2,4-Triazol-3-essigsäure-5-yl) |
| 5e | H | H | Cl | F | 5-F | -H | 2-(5-methyl-4-(2-carboxyphenyl)-4H-[1,2,4]-triazol-3-yl) |
| 6d | H | H | Cl | F | 5-F | 2-Chlor | 5-(5-Methyl-(1,2,4)-triazol-3-yl) |
| 6e | H | H | Cl | F | 5-F | 2-Chlor | 3-(4H-(1,2,4)-Triazol-3-yl) |
| 6f | H | H | Cl | F | 5-F | 2-OCF₃ | 4-(5-Hydroxy-1-H-(1,2,4)-triazol-3-yl) |
| 7c | H | H | Cl | F | H | 2-Chlor | 4-(1H-Tetrazol-5-yl) |
| 7d | H | H | Cl | F | 5-F | 2-Chlor | 4-(1H-Tetrazol-5-yl) |
| 7e | H | H | Cl | F | H | 2-OCF₃ | 4-(1 H-Tetrazol-5-yl) |
| 7f | H | H | Cl | F | 5-F | 2-OCF₃ | 4-(1 H-Tetrazol-5-yl) |
| 8c | H | H | Cl | F | H | 2-OMe | 4-(5-Hydroxy-(1,3,4)-oxdiazol-2-yl) |
| 8d | H | H | Cl | F | H | -H | 2-(5-Hydroxy-(1,3,4)-oxdiazol-2-yl) |
| 8e | H | H | Cl | F | 5-F | 2-OMe | 4-(5-Hydroxy-(1,3,4)-oxdiazol-2-yl) |
| 8f | H | H | Cl | F | 5-F | -H | 2-(5-Hydroxy-(1,3,4)-oxdiazol-2-yl) |
| 8g | H | H | Cl | F | H | -H | 2-(1,3,4-Oxdiazol-2-yl) |
| 8h | H | H | Cl | F | 5-F | -H | 2-(1,3,4-Oxdiazol-2-yl) |
| 9e | H | H | Cl | F | H | 2-OMe | 4-(5-Methoxy-2-oxo-1,3,4-oxdiazol-3-yl) |
| 9f | H | H | Cl | F | H | 2-Me | 4-(5-Methylamino-2-oxo-1,3,4-oxdiazol-3-yl) |
| 9g | H | H | Cl | F | H | 2-Chlor | 4-(5-Methyl-1,3,4-oxdiazol-2-yl) |
| 10c | H | H | Cl | F | 5-F | -H | 2-(5-Amino-1,3,4-oxdiazol-2-yl) |
| 11d | H | H | Cl | F | 5-F | 2-OCF₃ | 4-(5-Oxo-4,5-dihydro-(1,2,4)-oxdiazol-3-yl) |
| 11e | H | H | Cl | F | H | 2-OCF₃ | 4-(5-Oxo-4,5-dihydro-(1,2,4)-oxdiazol-3-yl) |
| 11f | H | H | Cl | F | H | -Cl | 4-(5-Oxo-4,5-dihydro-(1,2,4)-oxdiazol-3-yl) |
| 12c | H | H | Cl | F | H | 2-Me | 4-(3-Methyl-5-oxo-pyrazol-1-yl) |
| 13a | H | H | Cl | F | 5-F | -H | 2-(Benzimidazol-2-yl) |
| 13b | H | H | Cl | F | 5-F | -H | 2-(5-Carboxy-fur-2-yl) |
| 13c | H | H | Cl | F | H | -H | 2-(5-Carboxy-fur-2-yl) |
| 14e | H | H | Cl | F | 5-F | 2-Chlor | 5-(6-Methyl-5-oxo-4H-(1,2,4)-triazin-3-yl) |
| 14f | H | H | Cl | F | 5-F | 2-Chlor | 5-(5,6-Dimethyl-(1,2,4)-triazin-3-yl) |

**Tabelle II**

| | | | | | | | |
|---|---|---|---|---|---|---|---|
| Verbindungen der Formel Ia | | | | | | | |
| 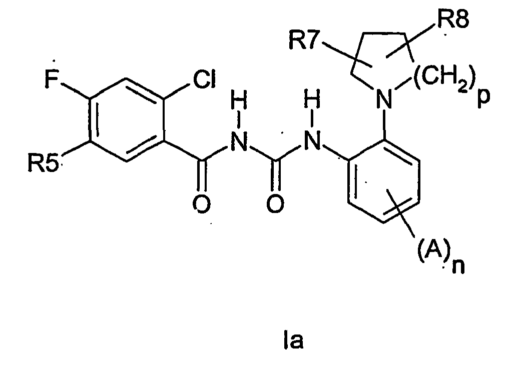 | | | | | | | |

| **Bsp.** | **R5** | **(A)ₙ** | **R7** | **(A)ₙ** | **R8** | **p** | **Fp.** |
|---|---|---|---|---|---|---|---|
| 15 | F | 5-F | H | H | 3-CONH₂ | 2 | 216,0 |
| 16 | F | 5-F | H | H | 4-COOH | 2 | 221,7 |
| 17 | F | 5-F | H | H | 3-COOH | 2 | 184,2 |
| 18 | H | 5-F | H | H | 3-COOH | 2 | 205,6 |
| 19 | H | 5-F | H | H | 3-CONH₂ | 2 | 205,0 |
| 20 | H | 5-F | H | H | 4-COOH | 2 | 230,7 |
| 21 | F | 5-SO₂Me | H | H | 3-COOH | 2 | Harz |
| 22 | H | 5-SO₂Me | H | H | 3-COOH | 2 | Harz |
| 23 | F | 5-CF₃ | H | H | 3-COOH | 2 | Harz |
| 24 | H | 5-CF₃ | H | H | 3-COOH | 2 | Harz |
| 25 | H | 4-Me | H | H | 3-COOH | 2 | 188,4 |
| 26 | F | 4-Me | H | H | 3-COOH | 2 | 184,9 |
| 27 | F | 5-Me | H | H | 3-COOH | 2 | 219,4 |
| 28 | H | 5-Me | H | H | 3-COOH | 2 | 228,3 |
| 29 | F | 5-Cl | H | H | 4-COOMe | 2 | 206,6 |
| 30 | H | 5-Cl | H | H | 4-COOMe | 2 | 211,2 |
| 31 | F | 5-Cl | H | H | 3-COOH | 2 | 203,6 |
| 32 | H | 5-Cl | H | H | 3-COOH | 2 | 215,8 |
| 33 | F | 5-COOMe | H | H | 3-COOH | 2 | 227,3 |
| 34 | H | 5-COOMe | H | H | 3-COOH | 2 | 219,4 |
| 35 | F | 5-F | H | H | 3-COOEt | 2 | Öl |
| 36 | H | 5-F | H | H | 3-COOEt | 2 | 145,2 |
| 37 | F | 5-F | H | H | 3-CONEt₂ | 2 | Öl |
| 38 | H | 5-F | H | H | 3-CONEt₂ | 2 | 178,2 |
| 39 | F | 5-F | H | H | 4-COOMe | 2 | 207,0 |
| 40 | H | 5-F | H | H | 4-COOMe | 2 | 187,2 |
| 41 | F | 5-COOMe | H | H | 4-COOMe | 2 | 221,1 |
| 42 | H | 5-COOMe | H | H | 4-COOMe | 2 | 205,3 |
| 43 | H | 5-CF₃ | H | H | 4-COOH | 2 | 219,9 |
| 44 | F | 5-SO₂Me | H | H | 4-COOMe | 2 | 231,5 |
| 45 | H | 5-SO₂Me | H | H | 4-COOMe | 2 | 228,2 |
| 46 | H | 5-CF₃ | H | H | 4-COOH | 2 | 206,3 |
| 47 | F | 5-CF₃ | H | H | 4-COOMe | 2 | Harz |
| 48 | F | H | H | H | 3-COOH | 1 | Harz |
| 49 | H | 5-CF₃ | H | H | 4-COOMe | 2 | Harz |
| 50 | F | 5-COOH | H | H | 3-COOH | 2 | 198,4 |
| 51 | F | 5-F | 4-Phenyl | H | 4-COOH | 2 | 241,6 |
| 52 | F | 4-COOH | H | H | 4-COOMe | 2 | 238,6 |
| 53 | F | 4-F | H | H | 4-COONa | 2 | 160,9 |
| 54 | F | H | H | H | 4-COOH | 2 | 221,6 |
| 55 | F | 5-Cl | H | H | 4-COOH | 2 | Harz |
| 56 | H | H | H | H | 4-COOH | 2 | 227,4 |
| 57 | F | 5-F | H | H | 4-COOH(Trissalz) | 2 | 180,6 |
| 58 | F | 5-CF₃ | H | H | 3-COOEt | 2 | 184,6 |
| 59 | F | 5-F | 4-Phenyl | H | 4-COOMe | 2 | 218,2 |
| 60 | F | 5-F | 4-Phenyl | H | 4-CONH₂ | 2 | 236,4 |

Die Verbindungen der Formel 1 zeichnen sich durch günstige Wirkungen auf den Lipid- und Kohlenhydratstoffwechsel aus, sie senken insbesondere den Blutzuckerspiegel und sind zur Behandlung von Typ 2 Diabetes, von Insulinresistenz, von Dyslipidämien und des metabolischen Syndroms / Syndrom X geeignet. Weiterhin sind die Verbindungen zur Prophylaxe und Behandlung von arteriosklerotischen Erscheinungen geeignet. Die Verbindungen können allein oder in Kombination mit weiteren Blutzucker senkenden Wirkstoffen eingesetzt werden.

### Die Wirksamkeit der Verbindungen wurde wie folgt getestet:

Glykogenphophorylase a Aktivitätstest

Der Effekt von Verbindungen auf die Aktivität der aktiven Form der Glykogenphosphorylase (GPa) wurde in der umgekehrten Richtung, durch Verfolgen der Glykogensynthese aus Glukose-1-Phosphat an Hand der Bestimmung der Freisetzung von anorganischem Phosphat, gemessen. Alle Reaktionen wurden als Doppelbestimmungen in Mikrotiterplatten mit 96-Vertiefungen (Half Area Plates, Costar Nr. 3696) durchgeführt, wobei die Änderung der Absorption auf Grund der Bildung des Reaktionsprodukts bei der weiter unten spezifizierten Wellenlänge in einem Multiskan Ascent Elisa. Reader (Lab Systems, Finnland) gemessen wurde. Um die GPa Enzymaktivität in der umgekehrten Richtung zu messen, wurde die Umwandlung von Glukose-1-Phosphat in Glykogen und anorganisches Phosphat nach der allgemeinen Methode von Engers et al. (Engers HD, Shechosky S, Madsen NB, Can J Biochem 1970 Jul;48(7):746-754) mit folgenden Modifikationen gemessen: Humane Glykogenphosphorylase a (zum Beispiel mit 0,76 mg Protein / ml (Aventis Pharma Deutschland GmbH), gelöst in Pufferlösung E (25 mM β-Glyzerophosphat, pH 7,0, 1 mM EDTA und 1 mM Dithiotreitol) wurde mit Puffer T (50 mM Hepes, pH 7,0, 100 mM KCI, 2,5 mM EDTA, 2,5 mM MgCl₂·6H₂O) und Zusatz von 5 mg/ml Glykogen auf eine Konzentration von 10 µg Protein/ml verdünnt. Prüfsubstanzen wurden als 10 mM Lösung in DMSO zubereitet und auf 50 µM mit Pufferlösung T verdünnt. Zu 10 µl dieser Lösung wurden 10 µl 37,5 mM Glukose, gelöst in Pufferlösung T und 5 mg/mL Glykogen, sowie 10 µl einer Lösung von humaner Glykogenphosphorylase a (10 µg Protein/ml) und 20 µl Glukose-1-Phosphat, 2,5 mM zugegeben. Der basale Wert der Glykogenphosphorylase a Aktivität in Abwesenheit von Prüfsubstanz wurde durch Zugabe von 10 µl Pufferlösung T (0,1 % DMSO) bestimmt. Die Mischung wurde 40 Minuten bei Raumtemperatur inkubiert und das freigesetzte anorganische Phosphat mittels der allgemeinen Methode von Drueckes et al. (al (Drueckes P, Schinzel R, Palm D, Anal Biochem 1995 Sep 1;230(1):173-177) mit folgenden Modifikationen gemessen: 50 µl einer Stop-Lösung von 7,3 mM Ammoniummolybdat, 10,9 mM Zinkacetat, 3,6 % Askorbinsäure, 0,9 % SDS werden zu 50 µl der Enzymmischung gegeben. Nach 60 Minuten Inkubation bei 45 °C wurde die Absorption bei 820 nm gemessen. Zur Bestimmung der Hintergrundsabsorption wurde in einem separaten Ansatz die Stop-Lösung unmittelbar nach Zugabe der Glukose-1-Phosphatlösung zugegeben.

Dieser Test wurde mit einer Konzentrationen von 10 µM der Prüfsubstanz durchgeführt, um die jeweilige Hemmung der Glykogenphosphorylase a in vitro durch die Prüfsubstanz zu bestimmen.

**Tabelle 2: Biologische Aktivität**

| **Bsp.** | **%Hemmung bei 10 µM** |
|---|---|
| | |
| 1c | 94 |
| 2c | 94 |
| 3 | 100 |
| 4d | 90 |
| 5e | 9 |
| 6d | 100 |
| 6e | 100 |
| 6f | 98 |
| 7c | 100 |
| 7d | 100 |
| 7e | 100 |
| 7f | 100 |
| 8c | 96 |
| 8d | 100 |
| 8e | 97 |
| 8f | 99 |
| 8g | 98 |
| 8h | 100 |
| 9e | 20 |
| 9f | 54 |
| 9g | 79 |
| 10c | 100 |
| 11d | 100 |
| 11e | 98 |
| 11f | 98 |
| 12c | 85 |
| 13a | 48 |
| 13b | 100 |
| 13c | 100 |
| 14e | 97 |
| 14f | 100 |
| 15c | 0,3 |
| 16c | 0,01 |
| 17 | 0,01 |
| 18 | 0,02 |
| 19 | 1,0 |
| 20 | 0,04 |
| 21 | 0,3 |
| 22 | 1,1 |
| 23 | 0,03 |
| 24 | 0,09 |
| 25 | 0,06 |
| 26 | 0,04 |
| 27 | 0,02 |
| 28 | 0,04 |
| 29 | 0,01 |
| 30 | 0,02 |
| 31 | 0,01 |
| 32 | 0,03 |
| 33 | 1,0 |
| 34 | 3,2 |
| 35 | 0,3 |
| 36 | 0,3 |
| 37 | 3,9 |
| 38 | 4,6 |
| 39 | 0,01 |
| 40 | 4,6 |
| 41 | 0,01 |
| 42 | 0,01 |
| 43 | 0,15 |
| 44 | 0,05 |
| 45 | 0,8 |
| 46 | 0,01 |
| 47 | 0,01 . |
| 48 | 0,01 |
| 49 | 0,01 |
| 50 | 0,01 |

Aus der Tabelle ist abzulesen, dass die Verbindungen der Formel 1 die Aktivität der Glykogenphosphorylase a hemmen und dadurch die Senkung des Blutzuckerspiegels bewirken.

## Patentansprüche

1. Verbindungen der Formel I, 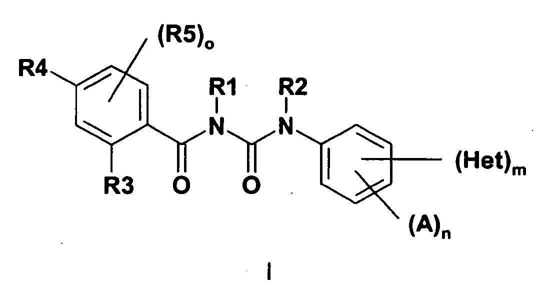 worin bedeuten
R1, R2 unabhängig voneinander H, (C₁-C₆)-Alkyl, wobei Alkyl mit OH, O-(C₁-C₄)-Alkyl, NH₂, NH-(C₁-C₄)-Alkyl oder N-[(C₁-C₆)-Alkyl]₂, substituiert sein kann, O-(C₁-C₆)-Alkyl, CO-(C₁-C₆)-Alkyl, COO-(C₁-C₆)-Alkyl, (C₁-C₆)-Alkylen-COOH oder (C₁-C₆)-Alkylen-COO-(C₁-C₆)-Alkyl;
R3, R4 unabhängig voneinander F, Cl, Br, OH, NO₂, CN, (C₁-C₆)-Alkyl, O-(C₁-C₄)-Alkyl, O-(C₂-C₆)-Alkenyl oder (C₂-C₆)-Alkinyl, wobei Alkyl, Alkenyl und Alkinyl mehrfach durch F, Cl oder Br substituiert sein können;
R5 H, F, Cl, Br, OH, NO₂, CN, (C₁-C₆)-Alkyl, O-(C₁-C₄)-Alkyl, CO-(C₁-C₆)-Alkyl, (C₀-C₆)-Alkylen-COOH, (C₀-C₆)-Alkylen-COO-(C₁-C₆)-Alkyl, SO₂-(C₁-C₆)-Alkyl, O-(C₂-C₆)-Alkenyl oder (C₂-C₆)-Alkinyl, wobei Alkyl, Alkenyl und Alkinyl mehrfach durch F, Cl oder Br substituiert sein können;
A H, F, Cl, Br, OH, NO₂, CN, (C₁-C₆)-Alkyl, CO-(C₁-C₆)-Alkyl, (C₀-C₆)-Alkylen-COOH, (C₀-C₆)-Alkylen-COO(C₁-C₆)-Alkyl, SO₂-(C₁-C₆)-Alkyl, (C₂-C₆)-Alkenyl, (C₂-C₆)-Alkinyl, O-(C₁-C₆)-Alkyl, S(O)₁₋₂-(C₁-C₆)-Alkyl-, NH-(C₁-C₆)-Alkyl, N-((C₁-C₆)-Alkyl)₂, COOH, COO-(C₁-C₆)-Alkyl, CONH₂, CONH-(C₁-C₆)-Alkyl, CON-((C₁-C₆)-Alkyl)₂, SO₂NH₂, SO₂NH-(C₁-C₆)-Alkyl, SO₂N-((C₁-C₆)-Alkyl)₂ oder NHCOR6, wobei Alkyl, Alkenyl und Alkinyl mehrfach durch F, Cl, Br, COOH, COO-(C₁-C₆)-Alkyl, CONH₂, CONH-(C₁-C₆)-Alkyl, CON-((C₁-C₆)-Alkyl)₂ oder OCO-(C₁-C₆)-Alkyl substituiert sein können;
R6 H, (C₁-C₆)-Alkyl, (C₃-C₇)-Cycloalkyl, (C₃-C₇)-Cycloalkyl-(C₁-C₄)-alkylen, (C₂-C₆)-Alkenyl, (C₂-C₆)-Alkinyl, (C₁-C₆)-Alkylen-COO-(C₁-C₆)-alkyl, (C₁-C₆)-Alkylen-CO-(C₁-C₆)-alkyl, (C₀-C₆)-Alkylen-COOH, , (C₁-C₆)-Alkylen-CONH₂, (C₆-C₁₀)-Aryl, (C₁-C₄)-Alkylen-(C₅-C₁₀)-aryl, Heteroaryl, (C₁-C₄)-Alkylen-heteroaryl oder CO-Heteroaryl, wobei Alkyl, Cycloalkyl, Alkylen, Alkenyl und Alkinyl mehrfach mit F, Cl, Br, O-(C₁-C₄)-Alkyl), COO-(C₁-C₄-Alkyl) oder N-((C₁-C₄)-Alkyl)₂ und wobei Aryl und Heteroaryl mehrfach mit F, Cl, Br, NO₂, CN, O-(C₁-C₄)-Alkyl), S-COO-(C₁-C₄-Alkyl), N-((C₁-C₄)-Alkyl)₂ oder (C₁-C₆)-Alkyl substituiert sein können;
n 0, 1, 2 oder 3;
m 1, 2, 3, 4 oder 5;
o 0, 1, 2 oder 3;
Het heterozyklischer 4- bis 7-gliedrigen Ring, der bis zu 4 Heteroatome N, O oder S als Ringglieder enthalten kann, wobei Pyrrol ausgenommen ist und wobei der heterozyklische Ring substituiert sein kann mit R7, R8 und R9;
R7, R8, R9 unabhängig voneinander H, F, Cl, Br, (C₁-C₆)-Alkyl , O-(C₁-C₆)-Alkyl, O-(C₂-C₆)-Alkenyl, O-(C₂-C₆)-Alkinyl, OH, Oxo, O-(C₁-C₆)-Alkyl, NH₂, NH-(C₁-C₆)-Alkyl, N-((C₁-C₆)-Alkyl)₂, COOH, CO-(C₁-C₆)-Alkyl, COO-(C₁-C₆)-Alkyl, CONH₂, CONH-(C₁-C₆)-Alkyl, CON-((C₁-C₆)-Alkyl)₂, (C₀-C₆)-Alkylen-Aryl oder (C₁-C₆)-Alkylen-COO-(C₁-C₆)-Alkyl; worin Alkyl, Alkenyl, Alkinyl, Alkylen und Aryl durch COOH, CONH₂, CONH-(C₁-C₆)-Alkyl, CON-((C₁-C₆)-Alkyl)₂, OCO-(C₁-C₆)-Alkyl, F, Cl, (C₁-C₆)-Alkyl oder O-(C₁-C₆)-Alkyl substituiert sein können;
und worin 2 der Reste R7, R8 und R9 gemeinsam einen an Het ankondensierten Ring bilden können;
sowie deren physiologisch verträgliche Salze.

2. Verbindungen der Formel I, gemäß Anspruch 1, **dadurch gekennzeichnet, dass** darin die Verbindungen die Struktur laa besitzen 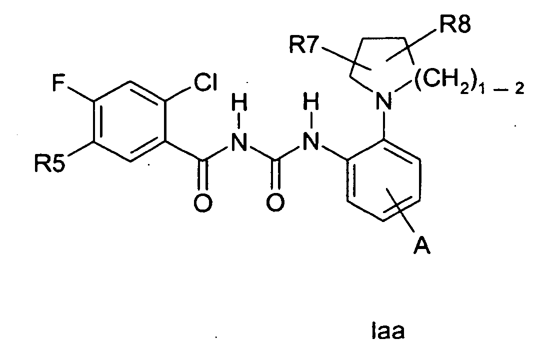 worin bedeuten
R5 H, F;
A H, F, Cl, (C₁-C₆)-Alkyl, CF₃, COO-(C₁-C₆)-Alkyl, COOH, SO₂-(C₁-C₆)-Alkyl;
R7 H, Phenyl;
R8 -(C=O)-X
X OH, O-(C₁-C₆)-Alkyl, NH₂, NH-(C₁-C₆)-Alkyl, N-((C₁-C₆)-Alkyl)₂;
sowie deren physiologisch verträgliche Salze.

3. Arzneimittel enthaltend eine oder mehrere der Verbindungen gemäß Anspruch 1 oder 2.

4. Arzneimittel enthaltend eine oder mehrere der Verbindungen gemäß Anspruch 1 oder 2und mindestens einen weiteren Wirkstoff.

5. Arzneimittel, gemäß Anspruch 4, **dadurch gekennzeichnet, dass** es als weiteren Wirkstoff eine oder mehrere Antidiabetika, hypoglykämischen Wirkstoffe, HMGCoA-Reduktase Inhibitoren, Cholesterinresorptionsinhibitoren, PPAR gamma Agonisten, PPAR alpha Agonisten, PPAR alpha/gamma Agonisten, Fibrate, MTP-Inhibitoren, Gallensäureresorptionsinhibitoren, CETP-Inhibitoren, polymere Gallensäureadsorber, LDL-Rezeptorinducer, ACAT-Inhibitoren, Antioxidantien, Lipoprotein-Lipase Inhibitoren, ATP-Citrat-Lyase Inhibitoren, Squalen synthetase inhibitoren, Lipoprotein(a) antagonisten, Lipase Inhibitoren, Insuline, Sulphonylharnstoffe, Biguanide, Meglitinide, Thiazolidindione, α-Glukosidase-Inhibitoren, auf den ATP-abhängigen Kaliumkanal der Betazellen wirkende Wirkstoffe, CART-Agonisten, NPY-Agonisten, MC4-Agonisten, Orexin-Agonisten, H3-Agonisten, TNF-Agonisten, CRF-Agonisten, CRF BP-Antagonisten, Urocortin-Agonisten, β3-Agonisten, MSH (Melanocyt-stimulierendes Hormon)-Agonisten, CCK-Agonisten, Serotonin-Wiederaufnahme-Inhibitoren, gemischte Sertonin- und noradrenerge Verbindungen, 5HT-Agonisten, Bombesin-Agonisten, Galanin-Antagonisten, Wachstumshormone, Wachstumshormon freisetzende Verbindungen, TRH-Agonisten, entkoppelnde Protein 2- oder 3-Modulatoren, Leptinagonisten, DA-Agonisten (Bromocriptin, Doprexin), Lipase/Amylase-Inhibitoren, PPAR-Modulatoren, RXR-Modulatoren oder TR-β-Agonisten oder Amphetamine enthält.

6. Verwendung der Verbindungen gemäß Anspruch 1 oder 2 zur Herstellung eines Medikamentes zur Blutzuckersenkung.

7. Verwendung der Verbindungen gemäß Anspruch 1 oder 2 zur Herstellung eines Medikamentes zur Behandlung des Typ 2 Diabetes.

8. Verwendung der Verbindungen gemäß Anspruch 1 oder 2 zur Herstellung eines Medikamentes zur Behandlung von Lipid- und Kohlenhydratstoffwechselstörungen.

9. Verwendung der Verbindungen gemäß Anspruch 1 oder 2 zur Herstellung eines Medikaments zur Behandlung arteriosklerotischer Erscheinungen.

10. Verwendung der Verbindungen gemäß Anspruch 1 oder 2 zur Herstellung eines Medikaments zur Behandlung von Insulin Resistenz.

11. Verfahren zur Herstellung der Verbindungen der Formel I, **dadurch gekennzeichnet dass** Harnstoffe der Formel 2 oder Anilinderivate der Formel 3 mit Aroyl-isocyanaten, mit reaktiven Säurederivaten, mit Säurechloriden oder mit Anhydriden, der Formel 4, 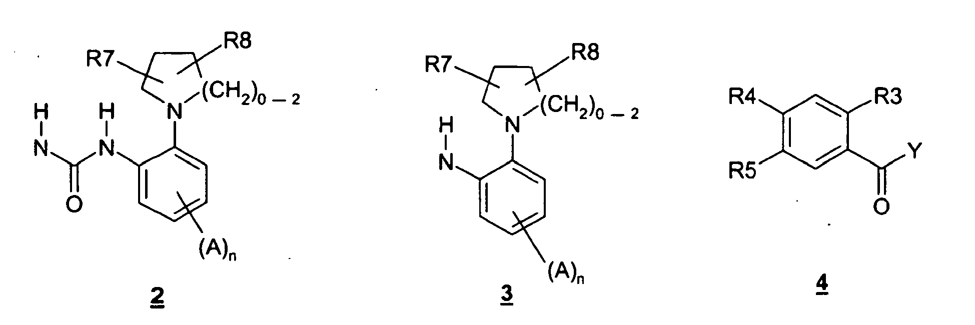 worin R3, R4, R5, R7, R8, A und X die oben angegebenen Bedeutungen haben, umgesetzt werden.

12. Verfahren zur Herstellung eines Arzneimittels enthaltend eine oder mehrere der Verbindungen gemäß Anspruch 1 oder 2, **dadurch gekennzeichnet, dass** der Wirkstoff mit einem pharmazeutisch geeigneten Träger vermischt wird und diese Mischung in eine für die Verabreichung geeignete Form gebracht wird.

## Claims

1. A compound of the formula I, 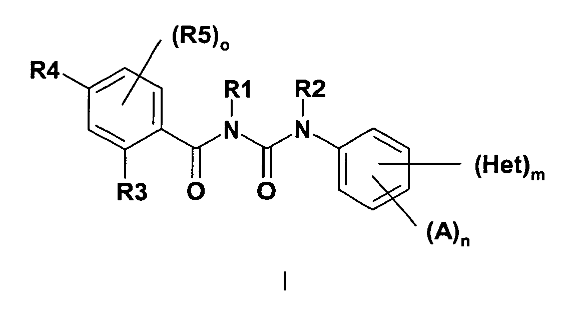 where
R1, R2 are each independently H, (C₁-C₆)-alkyl where alkyl may be substituted by OH, O-(C₁-C₄)-alkyl, NH₂, NH(C₁-C₄)-alkyl or N-[(C₁-C₆)-alkyl]₂, or are O-(C₁-C₆)-alkyl, CO-(C₁-C₆)-alkyl, COO-(C₁-C₆)-alkyl, (C₁-C₆)-alkylene-COOH or (C₁-C₆)-alkylene-COO-(C₁-C₆)-alkyl;
R3, R4 are each independently F, Cl, Br, OH, NO₂, CN, (C₁-C₆)-alkyl, O-(C₁-C₄)-alkyl, O-(C₂-C₆)-alkenyl or (C₂-C₆)-alkynyl, and alkyl, alkenyl and alkynyl may each be polysubstituted by F, Cl or Br;
R5 is H, F, Cl, Br, OH, NO₂, CN, (C₁-C₆)-alkyl, O-(C₁-C₄)-alkyl, CO-(C₁-C₆)-alkyl, (C₀-C₆)-alkylene-COOH, (C₀-C₆)-alkylene-COO-(C₁-C₆)-alkyl, SO₂-(C₁-C₆)-alkyl, O-(C₂-C₆)-alkenyl or (C₂-C₆)-alkynyl, and alkyl, alkenyl and alkynyl may each be polysubstituted by F, Cl or Br;
A is H, F, Cl, Br, OH, NO₂, CN, (C₁-C₆)-alkyl, CO-(C₁-C₆)-alkyl, (C₀-C₆)-alkylene-COOH, (C₀-C₆)-alkylene-COO(C₁-C₆)-alkyl, SO₂-(C₁-C₆)-alkyl, (C₂-C₆)-alkenyl, (C₂-C₆)-alkynyl, O-(C₁-C₆)-alkyl, S(O)₁₋₂-(C₁-C₆)-alkyl-, NH-(C₁-C₆)-alkyl, N-((C₁-C₆)-alkyl)₂, COOH, COO-(C₁-C₆)-alkyl, CONH₂, CONH-(C₁-C₆)-alkyl, CON-((C₁-C₆)-alkyl)₂, SO₂NH₂, SO₂NH-(C₁-C₆)-alkyl, SO₂N-((C₁-C₆)-alkyl)₂ or NHCOR6, and alkyl, alkenyl and alkynyl may each be polysubstituted by F, Cl, Br, COOH, COO-(C₁-C₆)-alkyl, CONH₂, CONH-(C₁-C₆)-alkyl, CON((C₁-C₆)-alkyl)₂ or OCO-(C₁-C₆)-alkyl;
R6 is H, (C₁-C₆)-alkyl, (C₃-C₇)-cycloalkyl, (C₃-C₇)-cycloalkyl-(C₁-C₄)-alkylene, (C₂-C₆)-alkenyl, (C₂-C₆)-alkynyl, (C₁-C₆)-alkylene-COO-(C₁-C₆)-alkyl, (C₁-C₆)-alkylene-CO-(C₁-C₆)-alkyl, (C₀-C₆)-alkylene-COOH, (C₁-C₆)-alkylene-CONH₂, (C₆-C₁₀)-aryl, (C₁-C₄)-alkylene-(C₆-C₁₀)-aryl, heteroaryl, (C₁-C₄)-alkylene-heteroaryl or CO-heteroaryl, and alkyl, cycloalkyl, alkylene, alkenyl and alkynyl may each be polysubstituted by F, Cl, Br, O(C₁-C₄-alkyl), COO-(C₁-C₄-alkyl) or N-((C₁-C₄)-alkyl)₂ and aryl and heteroaryl by F, Cl, Br, NO₂, CN, O-(C₁-C₄-alkyl), S-COO(C₁-C₄-alkyl), N-((C₁-C₄)-alkyl)₂ or (C₁-C₆)-alkyl;
n is 0, 1, 2 or 3;
m is 1, 2, 3, 4 or 5;
o is 0, 1, 2 or 3;
Het is heterocyclic 4- to 7-membered ring which may contain up to 4 N, O or S heteroatoms as ring members, with the exception of pyrrole, and the heterocyclic ring may be substituted by R7, R8 and R9;
R7, R8, and R9 are each independently H, F, Cl, Br, (C₁-C₆)-alkyl, O-(C₁-C₆)-alkyl, O-(C₂-C₆)-alkenyl, O-(C₂-C₆)-alkynyl, OH, oxo, O-(C₁-C₆)-alkyl, NH₂, NH-(C₁-C₆)-alkyl, N-((C₁-C₆)-alkyl)₂, COOH, CO-(C₁-C₆)-alkyl, COO-(C₁-C₆)-alkyl, CONH₂, CONH-(C₁-C₆)-alkyl, CON-((C₁-C₆)-alkyl)₂, (C₀-C₆)-alkylene-aryl or (C₁-C₆)-alkylene-COO-(C₁-C₆)-alkyl; where alkyl, alkenyl, alkynyl, alkylene and aryl may be substituted by COOH, CONH₂, CONH-(C₁-C₆)-alkyl, CON-((C₁-C₆)-alkyl)₂, OCO-(C₁-C₆)-alkyl, F, Cl, (C₁-C₆)-alkyl or O-(C₁-C₆)-alkyl;
and where 2 of the R7, R8 and R9 radicals together may form a ring fused onto Het;
and also its physiologically tolerated salts.

2. A compound of the formula I as claimed in claim 1, wherein the compound has the structure laa 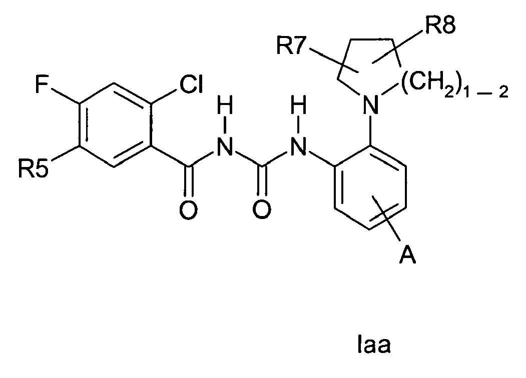 where
R5 is H, F;
A is H, F, Cl, (C₁-C₆)-alkyl, CF₃, COO-(C₁-C₆)-alkyl, COOH, SO₂-(C₁-C₆)-alkyl;
R7 is H, phenyl;
R8 is -(C=O)-X
X is OH, O-(C₁-C₆)-alkyl, NH₂, NH-(C₁-C₆)-alkyl, N-((C₁-C₆)-alkyl)₂;
and also its physiologically tolerated salts.

3. A pharmaceutical comprising one or more of the compounds as claimed in claim 1 or 2.

4. A pharmaceutical comprising one or more of the compounds as claimed in claim 1 or 2 and at least one further active ingredient.

5. A pharmaceutical as claimed in claim 4, which comprises, as a further active ingredient, one or more antidiabetics, hypoglycemic active ingredients, HMG-CoA reductase inhibitors, cholesterol absorption inhibitors, PPAR gamma agonists, PPAR alpha agonists, PPAR alpha/gamma agonists, fibrates, MTP inhibitors, bile acid absorption inhibitors, CETP inhibitors, polymeric bile acid adsorbents, LDL receptor inducers, ACAT inhibitors, antioxidants, lipoprotein lipase inhibitors, ATP-citrate-lyase inhibitors, squalene synthetase inhibitors, lipoprotein(a) antagonists, lipase inhibitors, insulins, sulfonylureas, biguanides, meglitinides, thiazolidinediones, α-glucosidase inhibitors, active ingredients acting on the ATP-dependent potassium channel of the beta cells, CART agonists, NPY agonists, MC4 agonists, orexin agonists, H3 agonists, TNF agonists, CRF agonists, CRF BP antagonists, urocortin agonists, β3 agonists, MSH (melanocyte-stimulating hormone) agonists, CCK agonists, serotonin reuptake inhibitors, mixed serotoninergic and noradrenergic compounds, 5HT agonists, bombesin agonists, galanin antagonists, growth hormones, growth hormone-releasing compounds, TRH agonists, uncoupling protein 2 or 3 modulators, leptin agonists, DA agonists (bromocriptine, Doprexin), lipase/amylase inhibitors, PPAR modulators, RXR modulators or TR-β agonists or amphetamines.

6. The use of the compounds as claimed in claim 1 or 2 for producing a medicament for blood sugar reduction.

7. The use of the compounds as claimed in claim 1 or 2 for producing a medicament for treating type 2 diabetes.

8. The use of the compounds as claimed in claim 1 or 2 for producing a medicament for treating lipid and carbohydrate metabolism disorders.

9. The use of the compounds as claimed in claim 1 or 2 for producing a medicament for treating arteriosclerotic symptoms.

10. The use of the compounds as claimed in claim 1 or 2 for producing a medicament for treating insulin resistance.

11. A process for preparing the compounds of the formula I, which comprises reacting ureas of the formula 2 or aniline derivatives of the formula 3 with aroyl isocyanates, with reactive acid derivatives, with acid chlorides or with anhydrides, of the formula 4 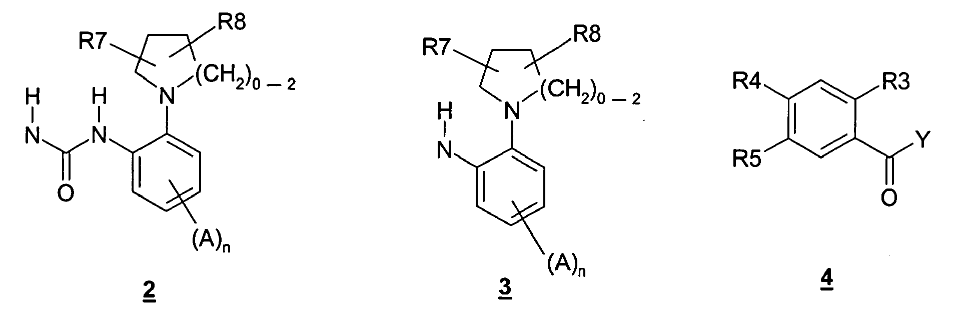 where R3, R4, R5, R7, R8, A and X are each as defined above.

12. A process for producing a pharmaceutical comprising one or more of the compounds as claimed in claim 1 or 2, which comprises mixing the active ingredient with a pharmaceutically suitable carrier and converting this mixture into a form suitable for administration.

## Revendications

1. Composés de formule I, 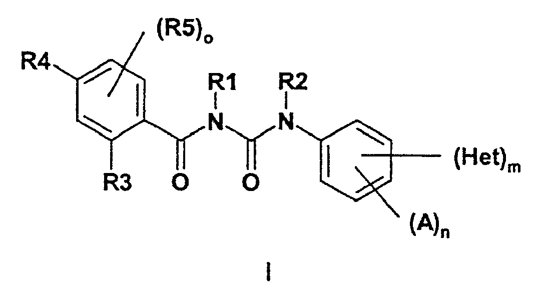 dans laquelle
R1, R2 sont chacun indépendamment H, alkyl(C₁-C₆), où alkyle peut être substitué par OH, O-alkyl(C₁-C₄), NH₂, NH-alkyl(C₁-C₄) ou N-(alkyl(C₁-C₆))₂, ou sont O-alkyl(C₁-C₆), CO-alkyl(C₁-C₆), COO-alkyl(C₁-C₆), alkylène(C₁-C₆)-COOH ou alkylène(C₁-C₆)-COO-alkyl(C₁-C₆) ;
R3, R4 sont chacun indépendamment F, Cl, Br, OH, NO₂, CN, alkyl(C₁-C₆), O-alkyl(C₁-C₄), O-alcényl(C₂-C₆) ou alcynyl(C₂-C₆), et alkyle, alcényle et alcynyle peuvent chacun être polysubstitués par F, Cl ou Br ;
R5 est H, F, Cl, Br, OH, NO₂, CN, alkyl(C₁-C₆), O-alkyl(C₁-C₄), CO-alkyl(C₁-C₆), alkylène(C₀-C₆)-COOH, alkylène(C₀-C₆)-COO-alkyl(C₁-C₆), SO₂-alkyl(C₁-C₆), O-alcényl(C₂-C₆) ou alcynyl(C₂-C₆), et alkyle, alcényle et alcynyle peuvent chacun être polysubstitués par F, Cl ou Br ; A est H, F, Cl, Br, OH, NO₂, CN, alkyl(C₁-C₆), CO-alkyl(C₁-C₆), alkylène(C₀-C₆)-COOH, alkylène(C₀-C₆)-COO-alkyl(C₁-C₆), SO₂-alkyl(C₁-C₆), alcényl(C₂-C₆), alcynyl(C₂-C₆), O-alkyl(C₁-C₆), S(O)₁₋₂-alkyl(C₁-C₆), NH-alkyl(C₁-C₆), N-(alkyl(C₁-C₆))₂, COOH, COO-alkyl(C₁-C₆), CONH₂, CONH-alkyl(C₁-C₆), CON-(alkyl(C₁-C₆))₂, SO₂NH₂, SO₂NH-alkyl(C₁-C₆), SO₂N-(alkyl(C₁-C₆))₂ ou NHCOR6, et alkyle, alcényle et alcynyle peuvent chacun être polysubstitués par F, Cl, Br, COOH, COO-alkyl(C₁-C₆), CONH₂, CONH-alkyl(C₁-C₆), CON(alkyl(C₁-C₆))₂ ou OCO-alkyl(C₁-C₆) ;
R6 est H, alkyl(C₁-C₆), cycloalkyl(C₃-C₇), cycloalkyl(C₃-C₇)-alkylène(C₁-C₄), alcényl(C₂-C₆), alcynyl(C₂-C₆), alkylène(C₁-C₆)-COO-alkyl(C₁-C₆), alkylène(C₁-C₆)-CO-alkyl(C₁-C₆), alkylène(C₀-C₆)-COOH, alkylène(C₁-C₆)-CONH₂, aryl(C₆-C₁₀), alkylène(C₁-C₄)-aryl(C₆-C₁₀), hétéroaryle, alkylène(C₁-C₄)-hétéroaryle ou CO-hétéroaryle, et alkyle, cycloalkyle, alkylène, alcényle et alcynyle peuvent chacun être polysubstitués par F, Cl, Br, O-(alkyle en C₁-C₄), COO-(alkyle en C₁-C₄) ou N-(alkyl(C₁-C₄))₂ et aryle et hétéroaryle par F, Cl, Br, NO₂, CN, O-(alkyle en C₁-C₄), S-COO-(alkyle en C₁-C₄), N-(alkyl(C₁-C₄))₂ ou alkyl(C₁-C₆) ;
n vaut 0, 1, 2 ou 3 ;
m vaut 1, 2, 3, 4 ou 5 ;
o vaut 0, 1, 2 ou 3 ;
Het est un cycle hétérocyclique de 4 à 7 chaînons pouvant contenir jusqu'à 4 hétéroatomes N, O ou S comme chaînons, à l'exception de pyrrole, et le cycle hétérocyclique peut être substitué par R7, R8 et R9 ;
R7, R8 et R9 sont chacun indépendamment H, F, Cl, Br, alkyl(C₁-C₆), O-alkyl(C₁-C₆), O-alcényl(C₂-C₆), O-alcynyl(C₂-C₆), OH, oxo, O-alkyl(C₁-C₆), NH₂, NH-alkyl(C₁-C₆), N-(alkyl(C₁-C₆))₂, COOH, CO-alkyl(C₁-C₆), COO-alkyl(C₁-C₆), CONH₂, CONH-alkyl(C₁-C₆), CON-(alkyl(C₁-C₆)₂, alkylène(C₀-C₆)-aryle ou alkylène(C₁-C₆)-COO-alkyl(C₁-C₆) ; où alkyle, alcényle, alcynyle, alkylène et aryle peuvent être substitués par COOH, CONH₂, CONH-alkyl(C₁-C₆), CON-(alkyl(C₁-C₆))₂, OCO-alkyl(C₁-C₆), F, Cl, alkyl(C₁-C₆) ou O-alkyl(C₁-C₆) ;
et où 2 parmi les radicaux R7, R8 et R9 peuvent former ensemble un cycle condensé sur Het ;
et également leurs sels physiologiquement tolérés.

2. Composés de formule I selon revendication 1, dans laquelle les composés possèdent la structure laa, 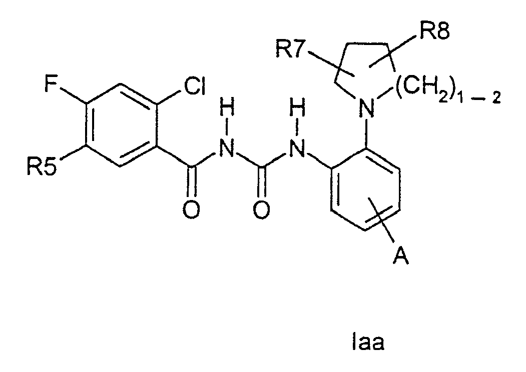 dans laquelle
R5 est H, F ;
A est H, F, Cl, alky/(C₁-C₆), CF₃, COO-alkyl(C₁-C₆), COOH, SO₂-alkyl(C₁-C₆) ;
R7 est H, phényle ;
R8 est -(C=O)-X ;
X est OH, O-alkyl(C₁-C₆), NH₂, NH-alkyl(C₁-C₆), N-(alkyl(C₁-C₆))₂ ;
et également leurs sels physiologiquement tolérés.

3. Produit pharmaceutique comprenant un ou plusieurs des composés selon la revendication 1 ou 2.

4. Produit pharmaceutique comprenant un ou plusieurs des composés selon la revendication 1 ou 2, et au moins un ingrédient actif supplémentaire.

5. Produit pharmaceutique selon la revendication 4, comprenant, comme ingrédient actif supplémentaire, un ou plusieurs parmi les anti-diabétiques, les ingrédients actifs hypoglycémiants, les inhibiteurs de la HMG-CoA réductase, les inhibiteurs de l'absorption du cholestérol, les agonistes du PPAR gamma, les agonistes du PPAR alpha, les agonistes du PPAR alpha/gamma, les fibrates, les inhibiteurs de la MTP, les inhibiteurs de l'absorption de l'acide biliaire, les inhibiteurs de la CETP, les chélateurs de l'acide biliaire polymères, les inducteurs du récepteur de LDL, les inhibiteurs de l'ACAT, les anti-oxydants, les inhibiteurs de lipoprotéine lipase, les inhibiteurs de l'ATP citrate lyase, les inhibiteurs de la squalène synthétase, les antagonistes de la lipoprotéine (a), les inhibiteurs de la lipase, les insulines, les sulfonylurées, les biguanides, les méglitinides, les thiazolidinediones, les inhibiteurs de l'α-glucosidase, les ingrédients actifs agissant sur le canal potassique ATP-dépendant des cellules bêta, les agonistes de CART, les agonistes du NPY, les agonistes du MC4, les agonistes de l'oréxine, les agonistes du H3, les agonistes du TNF, les agonistes du CRF, les antagonistes du CRF BP, les agonistes de l'urocortine, les agonistes β3, les agonistes de la MSH (hormone de stimulation des mélanocytes), les agonistes du CCK, les inhibiteurs de recaptage de la sérotonine, les composés mixtes sérotoninergiques et noradrénergiques, les agonistes de la 5HT, les agonistes de la bombésine, les antagonistes de la galanine, les hormones de croissance, les composés de libération de l'hormone de croissance, les agonistes de la TRH, les modulateurs de la protéine 2 ou 3 de découplage, les agonistes de la leptine, les agonistes du DA (bromocriptine, Doprexine), les inhibiteurs de lipase/amylase, les modulateurs du PPAR, les modulateurs du RXR ou les agonistes du TR-β ou les amphétamines.

6. Utilisation des composés selon la revendication 1 ou 2, destinée à la production d'un médicament pour la réduction du sucre sanguin.

7. Utilisation des composés selon la revendication 1 ou 2, destinée à la production d'un médicament pour le traitement du diabète de type 2.

8. Utilisation des composés selon la revendication 1 ou 2, destinée à la production d'un médicament pour le traitement des troubles du métabolisme des glucides et des lipides.

9. Utilisation des composés selon la revendication 1 ou 2, destinée à la production d'un médicament pour le traitement des symptômes artérioscléreux.

10. Utilisation des composés selon la revendication 1 ou 2, destinée à la production d'un médicament pour le traitement de l'insulino-résistance.

11. Procédé de préparation des composés de formule I, comprenant la réaction d'urées de formule 2 ou de dérivés d'aniline de formule 3 avec des aroyl-isocyanates, avec des dérivés d'acide réactifs, avec des chlorures d'acide ou avec des anhydrides, de formule 4, 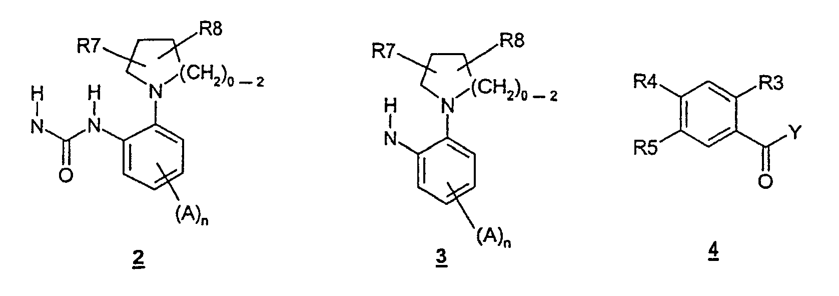 dans lesquelles R3, R4, R5, R7, R8, A et X sont chacun tels que définis ci-dessus.

12. Procédé de production d'un produit pharmaceutique comprenant un ou plusieurs des composés selon la revendication 1 ou 2, comprenant le mélange de l'ingrédient actif avec un véhicule pharmaceutiquement acceptable et la conversion de ce mélange en une forme convenable pour une administration.
